(19) Europäisches Patentamt
European Patent Office
Office européen des brevets

(11) Veröffentlichungsnummer : **0 059 684**
**B1**

(12) **EUROPÄISCHE PATENTSCHRIFT**

(45) Veröffentlichungstag der Patentschrift : **13.06.84**

(21) Anmeldenummer : **82810083.4**

(22) Anmeldetag : **22.02.82**

(51) Int. Cl.$^3$ : **C 07 C101/12**, C 07 C143/14, C 07 C143/78, C 07 D295/14, C 07 C103/82, C 07 C149/31, C 07 D295/22, C 11 D 3/42, D 06 L 3/12// C07C101/18

# (54) Amphotere Styrolderivate.

(30) Priorität : **26.02.81 CH 1295/81**

(43) Veröffentlichungstag der Anmeldung : **08.09.82 Patentblatt 82/36**

(45) Bekanntmachung des Hinweises auf die Patenterteilung : **13.06.84 Patentblatt 84/24**

(84) Benannte Vertragsstaaten : **AT BE CH DE FR GB IT LI NL SE**

(56) Entgegenhaltungen :
**EP-A- 0 019 078**
**EP-A- 0 019 702**
**CH-A- 543 490**
**DE-A- 1 443 342**
**US-A- 3 547 986**

(73) Patentinhaber : **CIBA-GEIGY AG**
**Postfach**
**CH-4002 Basel (CH)**

(72) Erfinder : **Meyer, Hans Rudolf, Dr.**
**Bollwerkstrasse 102**
**CH-4102 Binningen (CH)**
Erfinder : **Morf, Max, Dr.**
**Mittelfeldweg 30**
**CH-4124 Schönenbuch (CH)**

**EP 0 059 684 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue amphotere Styrolderivate, Verfahren zu deren Herstellung, deren Verwendung zum optischen Aufhellen von organischen Materialien, sowie Wasch- und Wäschebehandlungsmittel enthaltend solche amphotere Styrolderivate.

Aus den Europäischen Patentanmeldungen 19078 und 19702 sind kationische Distyrylbenzol- und Distyrylbiphenylverbindungen bekannt, welche auf Baumwolle hohe Aufhelleffekte in sauberen Waschflotten in Gegenwart von kationischen Weichmachern ergeben. Diese Effekte werden aber herabgesetzt durch die Anwesenheit grösserer Mengen von menschlichem Hautfett, das beim Waschen herausgelöst wird.

Der vorliegenden Erfindung lag die Aufgabe zugrunde, Aufheller zu finden, die diesen Nachteil nicht aufweisen.

Es wurde nun gefunden, dass amphotere Styrolderivate gegen den dem menschlichen Hautfett entsprechenden Schmutz unempfindlich sind.

Die erfindungsgemässen amphoteren Styrolderivate entsprechen der Formel

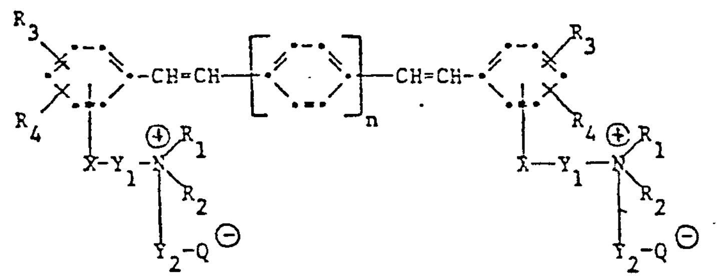

(1)

worin

X Sauerstoff, Schwefel, die direkte Bindung. —$SO_2N(R_5)$—, —$CON(R_5)$— oder —COO—,

$Y_1$ und $Y_2$ unabhängig voneinander $C_1$-$C_4$-Alkylen oder Hydroxypropylen,

$R_1$ und $R_2$ unabhängig voneinander $C_1$-$C_4$-Alkyl oder zusammen mit dem N-Atom einen Pyrrolidin-, Piperidin-, Hexamethylenimin- oder Morpholinring und $R_1$ zusammen mit $R_5$ auch einen Piperazinring,

$R_3$ und $R_4$ Wasserstoff, $C_{1-4}$-Alkyl, Chlor, $C_{1-4}$-Alkoxy, $C_{3-4}$-Alkenyl oder in o-Stellung zueinander zusammen eine Trimethylen- oder Tetramethylengruppe,

$R_5$ Wasserstoff, $C_{1-4}$-Alkyl, Cyanoäthyl oder zusammen mit $R_1$ einen Piperazinring,

Q —COO oder —$SO_3$ und

n die Zahl 1 oder 2

bedeuten.

Im Rahmen der erfindungsgemässen amphoteren Styrolderivate der Formel (1) sind besonders diejenigen zu erwähnen, welche der Formel

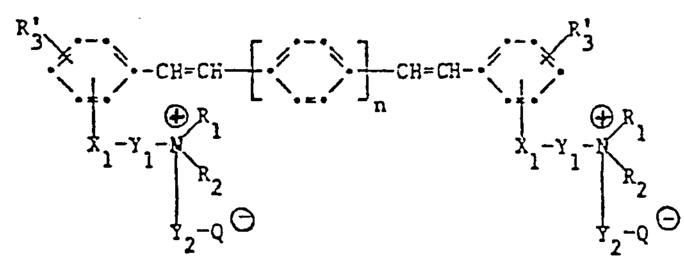

(2)

worin $R_1$, $R_2$, $Y_1$, $Y_2$, Q und n die oben angegebene Bedeutung haben und $X_1$ Sauerstoff, die direkte Bindung, —CONH— oder —COO— und $R_3'$ Wasserstoff, $C_{1-4}$-Alkyl, Methoxy oder Chlor bedeuten, entsprechen.

Bevorzugte amphotere Styrolderivate sind solche der Formel

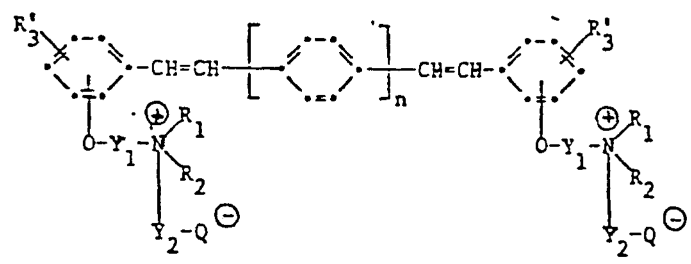

(3)

worin $R_1$, $R_2$, $R_3'$, $Y_1$, $Y_2$, Q und n die oben angegebene Bedeutung haben.

Besonders bevorzugt sind die amphoteren Styrolderivate der Formel

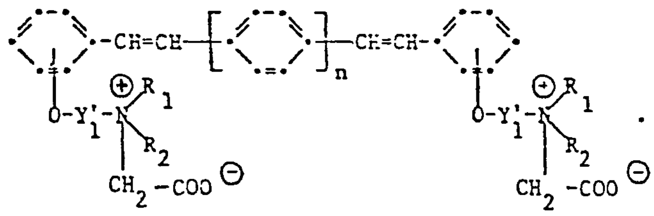

(4)

worin $Y_1'$ $C_{1-4}$-Alkylen bedeutet und $R_1$, $R_2$ und n die oben angegebene Bedeutung haben.

In den vorangehenden Formeln (1) bis (4) bedeuten vorzugsweise

X Sauerstoff, die direkte Bindung oder die —CONH-Gruppe,
$R_1$ und $R_2$ $C_{1-4}$-Alkyl,
$R_3$ Wasserstoff, Chlor, $C_{1-4}$-Alkyl oder $C_{1-4}$-Alkoxy,
$R_4$ und $R_5$ Wasserstoff,
$X_1$ Sauerstoff oder die direkte Bindung,
$Y_1$ und $Y_2$ $C_{1-4}$-Alkylen,
$R_3'$ Wasserstoff, Methyl, Methoxy oder Chlor,
Q die —COO-Gruppe und
$Y_2$ die —$CH_2$-Gruppe.

Ein weiterer Gegenstand der vorliegenden Erfindung ist ein Verfahren zur Herstellung der neuen amphoteren Styrolderivate, welches dadurch gekennzeichnet ist, dass man ein Styrolderivat der Formel

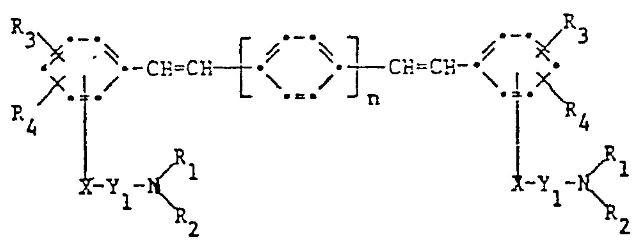

(5)

worin $R_1$, $R_2$, $R_3$, $R_4$, X, $Y_1$ und n die oben angegebene Bedeutung haben, mit einem Halogenid der Formel

$$Hal—Y_2—QH$$

worin $Y_2$ und Q die oben angegebene Bedeutung haben und Hal für Chlor oder Brom stehen oder wenn Q = —COO, mit dessen Salzen, Estern, Amiden, Nitrilen oder Lactonen oder wenn Q = —$SO_3$, mit dessen Salzen, Arylestern oder Sultonen oder wenn $Y_2$ = Hydroxypropylen auch mit den entsprechenden Epoxiden umsetzt und allfällig sich gebildete quaternäre Ammoniumhalogenide verseift. Unter Lactonen, Sultonen und Epoxiden sind die Dehydrohalogenierungsprodukte der Verbindungen der Formel Hal—$Y_2$—QH zu verstehen.

Die Betaine oder betainähnlichen Verbindungen existieren in amphoterer Form innerhalb eines weiten pH-Bereiches. Unter relativ stark sauren Bedingungen werden sie kationisch. Die Carbonsäuren können dann in Form ihrer Säureaddukte d. h. ihrer eine freie Carboxylgruppe enthaltenden Salze des Typus

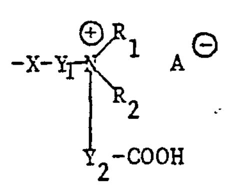

vorliegen, wobei $A^{\ominus}$ das Anion der Säure bedeutet. Doch ist die notwendige Azidität im allgemeinen ausserhalb des praktischen Bereiches für das Waschen von Geweben. Man erhält sie in einfacher Weise durch Zugabe einer anorganischen oder organischen Säure HA zur amphoteren Verbindung.

Als Säuren HA können z. B. Halogenwasserstoffsäuren, wie Chlor- und Bromwasserstoffsäure, Phosphorsäuredialkylester, Methanphosphonsäure, Ameisensäure, Essigsäure, Propionsäure, Glykolsäure, Milchsäure, Aepfelsäure, Weinsäure, Schleimsäure, Gluconsäure, Citronensäure, Laevulinsäure und Acrylsäure verwendet werden.

Bevorzugte Alkylierungsmittel sind Halogencarbonsäuren, deren Alkalisalze und Niederalkylester wie z. B. Chloressigsäure, Bromessigsäure, Chloressigsäure-natriumsalz, 2-Chloressigsäureäthylester, 2-Bromessigsäureäthylester, 2-Brompropionsäuremethylester, 4-Brombuttersäureäthylester ; Halogenalkansulfonate wie z. B. 2-Bromäthansulfonsäure Natriumsalz, 2-Bromäthansulfonsäurephenylester, 3-Chlor-2-hydroxy-propan-1-sulfosäure Natriumsalz, Brommethansulfosäure Natriumsalz ; Alkansultone wie 1,3-Propansulton, 1,4-Butansulton sowie Epoxyalkansulfonate wie 2,3-Epoxypropan-1-sulfosäure Natriumsalz. Letzteres wird vorteilhaft in Gegenwart einer Säure oder eines Salzes des verwendeten Amins eingesetzt.

Amphotere Sulfonsäuren der Formel (1), worin Q für $SO_3$ und $Y_2$ für Hydroxypropyl steht, erhält man auch durch Quaternierung von Verbindungen der Formel (5) mit Glycerindihalogenhydrinen oder mit Halogenwasserstoff und Epihalogenhydrinen zu Verbindungen der Formel

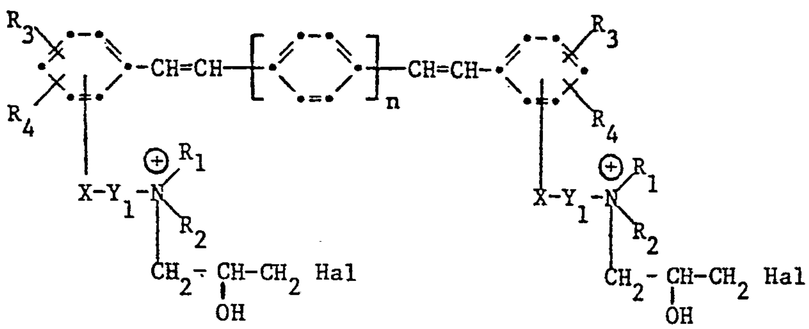

(6)

worin $R_1$, $R_2$, $R_3$, $R_4$, X, $Y_1$ und n die oben angegebene Bedeutung haben und Hal für Cl oder Br steht und anschliessende Umsetzung mit Alkali- oder Erdalkalibisulfiten gemäss P. Nikolaus, Fette, Seifen, Anstrichmittel *74*, 328-331 (1972).

Zu Verbindungen der Formel (1), worin Q für $SO_3$ und $Y_2$ für Propylen stehen, gelangt man auch durch Quaternierung von Verbindungen der Formel (5) mit Allylchlorid oder Allylbromid und anschliessende Umsetzung der erhaltenen Verbindungen der Formel

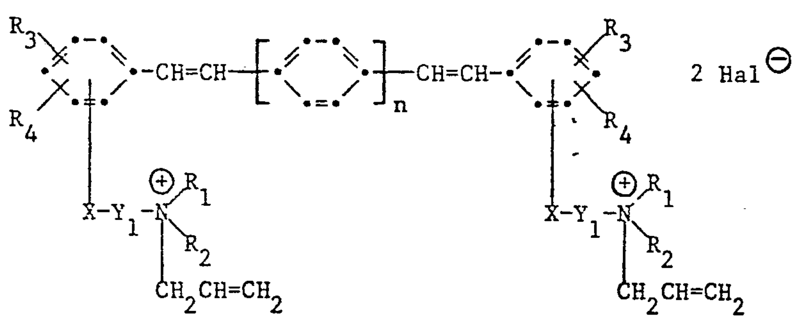

(7)

worin $R_1$, $R_2$, $R_3$, $R_4$, X, $Y_1$ und n die oben angegebene Bedeutung haben und Hal für Cl oder Br steht, mit Alkali- oder Erdalkalibisulfiten gemäss J. of American Oil Chem. Soc. *53*, 60-63 (1976) ; *54*, 294-296 (1977).

Verwendet man als Alkylierungsmittel Halogencarbonsäuren, dann fügt man zweckmässig mindestens die stöchiometrische Menge einer Base hinzu. Geeignete Basen sind Alkali- oder Erdalkalihydroxide oder Alkoholate wie Natriumhydroxid oder Natriummethylat, vor allem Alkali- und Erdalkalisalze schwacher Säuren wie Natriumcarbonat, Kaliumcarbonat, Calciumcarbonat oder Erdalkalioxide wie Magnesiumoxid in fein verteilter Form. Auch schwer quaternierbare tertiäre Amine wie Triisopropanolamin oder 2,6-Di-tert.-butylpyridin sind als Säureakzeptor brauchbar. Nach einem besonders günstigen Verfahren verwendet man direkt die Alkali- oder Erdalkalisalze der Halogencarbonsäuren wie z. B. das Natriumsalz der Chloressigsäure.

Verwendet man als Derivate der Halogencarbonsäuren deren Ester, Amide oder Nitrile, so entstehen primär die entsprechenden quaternären Ammoniumhalogenide, die sauer oder vorzugsweise alkalisch zu den Verbindungen der Formel (1) verseift werden. Besonders geeignete Halogencarbonsäurederivate sind die Ester, wie Chlor- oder Bromessigsäurealkylester mit 1 bis 4 C-Atomen in Alkylteil z. B. Chloressigsäuremethyl- oder äthylester. Beispiele für Amide und Nitrile sind Chloracetamid und Chloracetonitril. Auch Anhydride sowie Lactone vor allem β-Lactone der entsprechenden Hydroxycarbonsäuren wie Chloressigsäureanhydrid oder β-Propiolacton sind brauchbar. Anstelle der Halogencarbonsäuren können auch Tosylate der entsprechenden Hydroxycarbonsäuren als Alkylierungsmittel eingesetzt werden.

Die Reaktionstemperatur kann in weiten Grenzen zwischen etwa 20 °C und 150 °C, vorzugsweise 40-140 °C, gehalten werden, je nach der Reaktionsfähigkeit des verwendeten Quaternierungsmittels. Als

Reaktionsmedia in denen die Quaternierung vorgenommen werden kann, eignen sich im allgemeinen alle inerten Lösungsmittel. Bevorzugt sind solche, die das Ausgangsprodukt lösen und aus denen sich das Endprodukt sofort ausscheidet. Beispielsweise seien genannt: Aromatische Kohlenwasserstoffe wie Benzol, Toluol und Xylol; Halogenkohlenwasserstoffe wie Dichloräthan, Trichloräthan, Tetrachloräthylen, Chlorbenzol oder Dichlorbenzol, ferner auch Nitroverbindungen wie Nitromethan, Nitropropan, Nitrobenzol, Alkanole und offene oder cyclische Aether wie Methanol, Aethanol, Isopropanol, Butanol, Dibutyläther, Aethylenglykol, Aethylenglykolmonomethyläther, Aethylenglykolmonoäthyläther, Anisol oder Dioxan; Ketone wie Cyclohexanon oder Methyläthylketon; Fettsäureamide wie Dimethylformamid oder Dimethylacetamid; Sulfoxide wie Dimethylsulfoxid und Carbonsäure-ester oder -nitrile wie Essigester, Essigsäure-butylester, Acetonitril oder Methoxypropionitril. Bei in organischen Lösungsmitteln schwer löslichen Quaternierungsmitteln wird vorteilhaft in Wasser oder in Mischungen mit Wasser gearbeitet.

Zur Verseifung der erhaltenen quaternären Ammoniumhalogenide verwendet man wässrige Säuren, z. B. katalytische Mengen vor Mineralsäuren wie Salzsäure oder Schwefelsäure oder vorzugsweise Basen wie Alkali- oder Erdalkalihydroxide in mindestens stöchiometrischen Mengen in Wasser und/oder in mit Wasser mischbaren organischen Lösungsmitteln wie z. B. Alkoholen bei Temperaturen von etwa 40°-110 °C.

Die Quaternierung und Verseifung kann gewünschtenfalls auch im Eintopfverfahren durchgeführt werden.

Eine weitere Methode zur Herstellung der amphoteren Styrolderivate der Formel (1) besteht darin, dass man Verbindungen der Formel

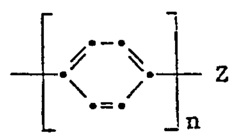

(8)

mit Aldehyden der Formel

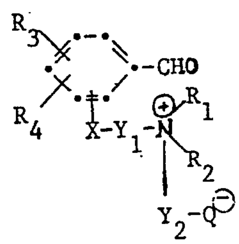

(9)

im Molekularverhältnis 1 : 2 in an sich bekannter Weise in Gegenwart einer starken Base umsetzt, in welchen Formeln X, $Y_1$, $Y_2$, $R_1$, $R_2$, $R_3$, $R_4$, Q und n die oben angegebene Bedeutung haben und Z eine Gruppierung der Formel

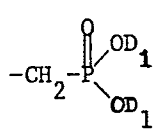 , 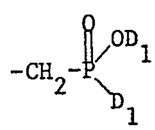

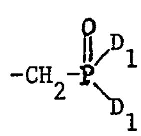 oder 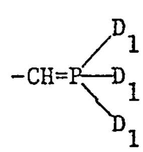

bedeutet, worin $D_1$ einen unsubstituierten oder substituierten Alkyl-, Aryl-, Cycloalkyl- oder Aralkylrest darstellt.

Die Menge der einzusetzenden Base ist mindestens 1 Aequivalent pro Mol Aldehyd. Anstelle der Verbindungen der Formel (9) können auch deren Säureaddukte der Formel

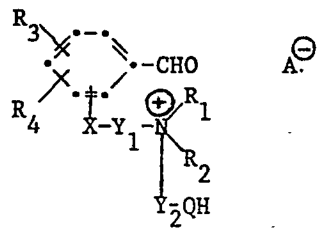

(10)

eingesetzt werden, worin X, $Y_1$, $Y_2$, $R_1$, $R_2$, $R_3$, $R_4$ und A die oben angegebene Bedeutung haben und Q vorzugsweise —COO— bedeutet. In diesem Fall werden mindestens 2 Aequivalente Base pro Mol Aldehyd benötigt.

Man führt die Kondensation vorteilhaft in indifferenten Lösungsmitteln durch. Als Beispiele hierfür seien Kohlenwasserstoffe wie Toluol und Xylol oder Alkohole wie Methanol, Aethanol, Isopropanol, Butanol, Glykole, Glykoläther wie 2-Methoxyäthanol, Hexanole, Cyclohexanol und Cyclooctanol, ferner Aether wie Diisopropyläther, Tetrahydrofuran und Dioxan sowie Dimethylsulfoxid, Formamid und N-Methylpyrrolidon genannt. Besonders geeignet sind polare organische Lösungsmittel wie Dimethylformamid und Dimethylsulfoxid. Auch in wässriger Lösung lassen sich einige der Umsetzungen durchführen.

Die Temperatur, bei welcher die Umsetzung durchgeführt wird, kann in weiten Grenzen schwanken. Sie wird bestimmt:

α) durch die Beständigkeit des verwendeten Lösungsmittels gegenüber den Reaktionsteilnehmern, insbesondere gegenüber den stark basischen Alkaliverbindungen,

β) durch die Reaktivität der Kondensationspartner und

γ) durch die Wirksamkeit der Kombination Lösungsmittel-Base als Kondensationsmittel.

In der Praxis kommen hiernach im allgemeinen Temperaturen zwischen etwa 10 und 100 °C in Betracht. Wenn Dimethylformamid als Lösungsmittel verwendet wird, liegt der Temperatur-Vorzugsbereich bei 20 bis 60 °C.

Als stark basische Alkaliverbindungen kommen vor allem die Hydroxide, Amide und Alkoholate (vorzugsweise 1 bis 4 Kohlenstoffatome enthaltender Alkohole) der Alkalimetalle in Betracht, wobei aus ökonomischen Gründen solche des Lithiums, Natriums und Kaliums von vorwiegendem Interesse sind. Es können jedoch grundsätzlich und in besonderen Fällen auch Alkalisulfide und -carbonate, Arylalkaliverbindungen wie z. B. Phenyl-lithium oder stark basische Amine (einschliesslich Ammoniumbasen), z. B. Trialkylammoniumhydroxyde, mit Erfolg verwendet werden.

Die Ausgangsprodukte der Formeln (9) und (10) erhält man durch Quaternieren der Verbindungen der Formel

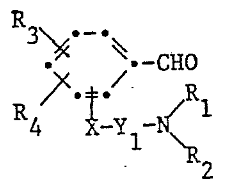

(11)

worin X, $Y_1$, $R_1$, $R_2$, $R_3$ und $R_4$ die oben angegebene Bedeutung haben, mit einem Halogenid der Formel Hal—$Y_2$—QH, deren Salzen, Estern oder Lactonen analog derjenigen der Styrolderivate der Formel (5) und Verseifung allfällig entstandener quaternärer Ammoniumhalogenide. Die Verseifung erfolgt in diesem Fall mit der stöchiometrischen Menge einer Base bzw. vorzugsweise mit einer Säure H—Hal des verwendeten Halogenids. Die amphoteren Verbindungen der Formel (9) erhält man auch durch Neutralisation der durch saure Verseifung entstandenen Produkte der Formel (10).

Nach den beschriebenen Verfahrensweisen können konzentrierte, stabile wässrige Lösungen der neuen amphoteren Styrolderivate erhalten werden, welche neben Wasser noch zusätzliche Lösungsmittel wie z. B. einen Alkohol aus der vorangehenden Ester-Hydrolyse und das Verseifungsprodukt des überschüssigen Quaternierungsmittels, z. B. Glykolsäure sowie Alkalihalogenid enthalten.

Die Erfindung betrifft somit auch lagerstabile, konzentrierte wässrige Lösungen von amphoteren Styrolderivaten der Formel (1), welche dadurch gekennzeichnet sind, dass sie

a) 0,1 bis 50 Gewichtsprozent eines amphoteren Styrolderivates der Formel

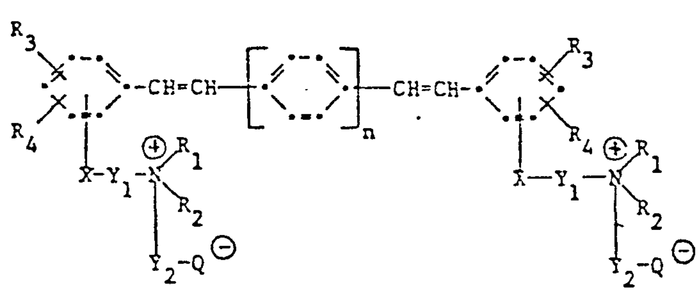

(1)

worin

X Sauerstoff, Schwefel, die direkte Bindung, —$SO_2N(R_5)$—, —$CON(R_5)$— oder —COO—,

$Y_1$ und $Y_2$ unabhängig voneinander $C_1$-$C_4$-Alkylen oder Hydroxypropylen,

$R_1$ und $R_2$ unabhängig voneinander $C_1$-$C_4$-Alkyl oder zusammen mit dem N-Atom einen Pyrrolidin-, Piperidin-, Hexamethylenimin- oder Morpholinring und $R_1$ zusammen mit $R_5$ auch einen Piperazinring,

$R_3$ und $R_4$ Wasserstoff, $C_{1-4}$-Alkyl, Chlor, $C_{1-4}$-Alkoxy, $C_{3-4}$-Alkenyl oder in o-Stellung zu einander zusammen eine Trimethylen- oder Tetramethylengruppe,

$R_5$ Wasserstoff, $C_{1-4}$-Alkyl, Cyanoäthyl oder zusammen mit $R_1$ einen Piperazinring,

Q —COO oder —$SO_3$ und

n die Zahl 1 oder 2

bedeuten.

b) 0 bis 20 Gewichtsprozent des sich aus dem verwendeten Quaternisierungs- und Verseifungsmittel gebildeten Alkalihalogenids und

c) Wasser enthalten.

Die erfindungsgemässen Lösungen eignen sich auch zum Aufhellen von Polyacrylnitrilfasern im Gelzustand. Sie sind über längere Zeiträume hinweg bei Lagerung unter den auftretenden schwankenden Temperaturen chemisch und physikalisch beständig, d. h. es tritt weder eine Zersetzung noch ein Ausscheiden des festen Produktes ein, auch nicht nach beliebigem Verdünnen mit Wasser. Durch Verdunsten angetrocknete Salze bleiben weiterhin wasserlöslich.

Die vorstehend definierten neuen Verbindungen zeigen in gelöstem oder feinverteiltem Zustande eine mehr oder weniger ausgeprägte Fluoreszenz. Sie können zum optischen Aufhellen der verschiedensten synthetischen, halbsynthetischen oder natürlichen organischen Materialien oder Substanzen, welche solche organische Materialien enthalten, verwendet werden.

Die optisch aufzuhellenden organischen Materialien können den verschiedenartigsten Verarbeitungszuständen (Rohstoffe, Halbfabrikate oder Fertigfabrikate) angehören. Den erfindungsgemäss anzuwendenden Verbindungen kommt u. a. Bedeutung für die Behandlung von textilen organischen Materialien, insbesondere textilen Geweben, zu. In Abhängigkeit vom verwendeten Aufheller-Verbindungstyp kann es sich als vorteilhaft erweisen, in neutralem oder alkalischem oder saurem Bade zu arbeiten.

Die neuen optischen Aufhellmittel gemäss vorliegender Erfindung können auch zum optischen Aufhellen von Papiermassen, unter anderem in Gegenwart von z. B. kationischen Retentionsmitteln und anderen Zusatzstoffen eingesetzt werden.

Die neuen optischen Aufhellmittel gemäss vorliegender Erfindung können in folgenden Anwendungsformen eingesetzt werden :

a) Mischungen mit Farbstoffen (Nuancierung) oder Pigmenten (Farb- oder insbesondere z. B. Weisspigmenten) oder als Zusatz zu Färbebädern ;

b) in Mischungen mit Netzmitteln, Weichmachern, Quellmitteln oder Antioxydantien ;

c) in Kombination mit verschiedenen Textilveredlungsverfahren, wie z. B. Flammfest-, Weichgriff-, Schmutzablöse (« anti-soiling »)- oder Antistatisch-Ausrüstungen oder antimikrobiellen Ausrüstungen ;

d) Einarbeiten der optischen Aufhellmittel in polymere Trägermaterialien (Polymerisations-, Polykondensations- oder Polyadditionsprodukte) in gelöster oder dispergierter Form für Anwendungen z. B. in Beschichtungs-, Imprägnier- oder Bindemitteln (Lösungen, Dispersionen, Emulsionen) für Textilien, Vliese, Papier, Leder ;

e) als Zusätze zu den verschiedensten industriellen Produkten, um dieselben marktfähiger zu machen (z. B. Aspektverbesserung von Seifen, Waschmitteln und Textilbehandlungsmitteln, Pigmenten) ;

f) in Kombination mit anderen, optisch aufhellend wirkenden Substanzen ;

g) in Spinnbadpräparationen, d. h. als Zusätze zu Spinnbädern, wie sie zur Gleitfähigkeitsverbesserung für die Weiterverarbeitung von Synthese-Fasern verwendet werden, oder aus einem speziellen Bad vor der Verstreckung der Faser, z. B. als Nachbehandlung von nassversponnenen Polyacrylfasern im sog. Gelzustand ;

h) für verschiedene Zwecke photographischer Art, wie z. B. für elektrophotographische Reproduktion oder Supersensibilisierung ;

i) je nach Substitution als Laser-Farbstoffe.

Wird das Aufhellverfahren mit Textil-Behandlungs- oder Veredlungsmethoden kombiniert, so kann die kombinierte Behandlung in vielen Fällen vorteilhafterweise mit Hilfe entsprechender beständiger Präparate erfolgen, welche die optisch aufhellenden Verbindungen in solcher Konzentration enthalten, dass der gewünschte Aufhelleffekt erreicht wird.

Die Menge der erfindungsgemäss zu verwendenden neuen optischen Aufheller, bezogen auf das optisch aufzuhellende Material, kann in weiten Grenzen schwanken. Schon mit sehr geringen Mengen, in gewissen Fällen z. B. solchen von 0,000 1 Gewichtsprozent, kann ein deutlicher und haltbarer Effekt erzielt werden. Es können aber auch Mengen bis zu etwa 0,8 Gewichtsprozent und gegebenenfalls bis zu etwa 2 Gewichtsprozent zur Anwendung gelangen. Für die meisten praktischen Belange sind vorzugsweise Mengen zwischen 0,000 5 und 0,5 Gewichtsprozent von Interesse.

Aus verschiedenen Gründen ist es oft zweckmässig, die Aufheller nicht als solche, d. h. rein einzusetzen, sondern vermischt mit den verschiedensten Hilfs- und Coupiermitteln.

Die neuen optischen Aufhellmittel eignen sich besonders als Zusätze für Waschbäder oder zu Gewerbe- und Haushaltswasch- und Wäsche- nachbehandlungsmitteln, wobei sie in verschiedener Weise zugesetzt werden können. Zu Waschbädern werden sie zweckmässig in Form ihrer Lösungen in Wasser

oder organischen Lösungsmitteln oder auch in feiner Verteilung als wässrige Dispersionen zugegeben. Zu Haushalt- oder gewerblichen Waschmitteln werden sie vorteilhaft in irgendeiner Phase des Herstellungsprozesses der Waschmittel zugesetzt. Die Zugabe kann sowohl in Form einer Lösung oder Dispersion in Wasser oder anderen Lösungsmitteln als auch ohne Hilfsmittel als trockenes Aufhellerpulver erfolgen. Man kann die Aufhellmittel beispielsweise in den waschaktiven Substanzen lösen oder mit denselben vermischen, verkneten oder vermahlen und so dem fertigen Waschmittel zumischen. Sie können jedoch auch gelöst oder vordispergiert auf das fertige Waschmittel aufgesprüht werden.

Die erfindungsgemässen Verbindungen können auch im Nachspülbad, wie es zur blossen Verleihung von Weichgriff, antistatischen Eigenschaften, Antisoileffekten, Duftnoten usw. üblich ist, eingesetzt werden. Insbesondere eignen sie sich für den Einsatz in Wäschenachbehandlungsmitteln, welche kationische Weichmacher enthalten.

Die vorliegende Erfindung betrifft daher auch ein Waschmittel, welches vorzugsweise in flüssiger Form vorliegt und welches neben den neuen amphoteren Styrolderivaten und den üblichen Zusätzen noch nicht-ionogene Tenside und kationische Textilweichmacher enthält.

Als nicht-ionogene Tenside kommen die handelsüblichen in Betracht, z. B. die wässerlöslichen Produkte die aus der Addition eines Alkylenoxides bzw. einer äquivalenten Verbindung mit einem reaktiven Wasserstoff einer hydrophoben Verbindung erhalten werden. Die hydrophoben organischen Produkte können Heterocyclen und besonders Aliphaten oder Aromaten sein. Bevorzugt sind höhere aliphatische Alkohole und Alkylphenole, wobei aber auch andere, wie z. B. Carbonsäuren, Carboxamide, Mercaptane, Sulfamide usw. verwendet werden können. Bevorzugte nicht-ionogene Verbindungen sind die Additionsprodukte von Aethylenoxid mit höheren aliphatischen Alkoholen mit 6 bis 50 und mehr C-Atomen. Die Menge Aethylenoxid kann sich in weiteren Grenzen bewegen, aber im allgemeinen werden zumindest 5 Mol Aethylenoxid pro Mol hydrophober Substanz gebraucht. Anstelle von Aethylenoxid können ganz oder teilweise andere niedere Alkylenoxide, wie z. B. Propylenoxid und Butylenoxid verwendet werden. Als weitere nicht-ionogene verwendbare Verbindungen kommen in Betracht :

a) Polyoxyalkylenester organischer Säuren wie höherer Fettsäuren, Harzsäuren, Tallölsäuren und Säuren der Oxydationsprodukte des Erdöls, deren Ester in der Regel im Säureteil 10 bis 22 C-Atome aufweisen und ca. 12 bis ca. 30 Mol Aethylenoxid oder dessen Aequivalent enthalten ;

b) Alkylenoxidaddukte höherer Fettsäureamide, wobei der Fettsäureanteil in der Regel 8 bis 22 C-Atome aufweisen und mit 10 bis 50 Mol Aethylenoxid kondensiert ist. Die entsprechenden Carboxamide und Sulfamide können ebenfalls als weitgehend äquivalent verwendet werden.

In der Herstellung von flüssigen konzentrierten Waschmitteln werden als nicht-ionogene Tenside vorzugsweise oxalkylierte höhere aliphatische Alkohole verwendet, wobei die Fettalkohole mindestens 6 und vorzugsweise mindestens 8 C-Atome vorweisen. Bevorzugte Alkohole sind Lauryl-, Myristyl-, Cetyl-, Stearyl- und Oleylalkohol, welche mit mindestens 6 Mol Aethylenoxid kondensiert werden. Als typisches nicht-ionogenes Produkt sei das Additionsprodukt von einem aliphatischen Alkohol mit 12-13 C-Atomen mit ca. 6,5 Mol Aethylenoxid erwähnt. Die entsprechenden Alkylmercaptane sind, nach Kondensation mit Aethylenoxid, ebenfalls als nicht-ionogene Tenside verwendbar.

Die alkoxylierten höheren aliphatischen Alkohole sind besonders für Haushaltswaschmittel geeignet, da sie leicht biologisch abbaubar sind und gut mit kationischen Tensiden und Textilweichmachern und den übrigen Zusätzen verträglich sind.

Von den kationischen Textilweichmachern eignen sich besonders quaternäre Derivate des Ammoniaks und/oder des Imidazolins mit 2 langkettigen aliphatischen Resten, wie z. B. das 1-Methyl-1-oleylamidoäthyl-2-oleyl-imidazolinium · $X^{\ominus}$, das 1-Methyl-2-talgamidoäthyl-2-talg-imidazolinium · $X^{\ominus}$, das Di-talg-dimethyl-ammonium · $X^{\ominus}$ und eine Verbindung der Formel

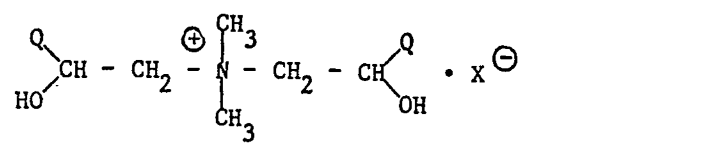

worin Q $C_{14-16}$-Alkyl bedeutet und $X^{\ominus}$ für ein Chlorid-, Bromid-, Methylsulfat-, Aethylsulfat, Methan-, Aethan- oder Toluolsulfonat-anion steht.

Die erfindungsgemäss verwendbaren quaternären Textilweichmacher und besonders die vorstehend genannten verleihen dem Gewebe einen weichen und flaumigen Griff und gleichzeitig eine gute Wiederanfeuchtbarkeit. Diese Textilweichmacher sind substantiv zum Gewebe und tragen zur Verminderung der statischen Aufladung und der Neigung zum Knittern bei, so dass das Gewebe leichter gebügelt werden kann und angenehmer zu tragen ist.

Das flüssige Medium für die erfindungsgemässen Waschmittel ist wässerig und kann aus Wasser allein oder aus Wasser und zusätzlichen Lösungsmitteln für gewisse Zusätze bestehen. Die zusätzlichen Lösungsmittel können bis zu 20, vorzugsweise bis 15 % des gesamten Lösungsmittelanteils ausmachen. Als solche kommen in Betracht : niedere Alkanole oder ein niederes Diol oder Polyol, wie z. B. Aethanol, Isopropanol, Aethylenglykol, Propylenglykol und Glycerin. Auch veräthertes Polyole, wie Diäthylenglykol, Aethylenglykoldiäthyläther und Aethylenglykolmonoäthyläther können als zusätzliche Lösungsmittel verwendet werden.

Das erfindungsgemässe flüssige Waschmittel kann verschiedene ausgewählte verträgliche Zusätze enthalten, wie Schmutzsuspendiermittel oder Vergrauungsinhibitoren, z. B. Polyvinylalkohol, Hydroxypropylmethylcellulose ; Schauminhibitoren, Konservierungsmittel, z. B. Natriumbenzoat ; UV-Absorber und Parfüme. Diese werden selbstverständlich so gewählt, dass sie mit den Hauptkomponenten des Waschmittels verträglich sind.

Die nicht-ionogenen Tenside werden in Mengen von 10 bis 70 Gew.-%, vorzugsweise 60 Gewichts-% eingesetzt. Die Konzentration des Textilweichmachers beträgt 1 bis 30 Gewichts-%, vorzugsweise 6 bis 21 Gewichts-%. Das wässrige Lösungsmittel, vorzugsweise Wasser, das noch mono-, di- und mehrwertige Alkohole und ähnliche Lösungsmittel enthalten kann, beträgt 5 bis 60 Gewichts-%. Das flüssige oder pulverförmige, fertige Waschmittel enthält die erfindungsgemässen Verbindungen in Mengen von 0,005 bis 3 Gewichts-%. Der Gehalt an den übrigen Hilfsmitteln beträgt vorzugsweise weniger als 5 Gewichts-% des Waschmittels, da die Verwendung von grösseren Mengen die Eigenschaften flüssiger Waschmittel beeinflussen kann. Obschon die bevorzugte anmeldungsgemässe waschaktive Zubereitung eine stabile, klare Flüssigkeit ist, kann man ihr ein verträgliches Trübungsmittel hinzugeben, um einen opaken Aspekt hervorzurufen.

Das erfindungsgemässe Waschmittel kann in weichem oder angemessen hartem Wasser bei höherer Temperatur zur Anwendung gelangen. Dieses Waschmittel kann auch zum Waschen von Textilien in sehr hartem Wasser bei tieferer Temperatur verwendet werden. Die Wasserhärte kann deshalb von 0 bis über 300 ppm, berechnet als Calciumcarbonat schwanken und die Waschtemperatur kann 4 bis 60 °C betragen.

Das erfindungsgemässe Waschmittel löst sich sehr leicht in kaltem oder warmem Waschwasser, reinigt gründlich, eliminiert die statische Aufladung und macht die Wäsche weich, ohne sie zu hydrophobieren. Das bevorzugte Waschmittel liegt als eine klare, stabile Flüssigkeit vor, die ihre Aktivität und Uniformität über eine längere Zeitspanne behält. Zur Herstellung von klaren flüssigen Waschmitteln kann die Konzentration der Aktivsubstanzen nur innerhalb gewisser Grenzen variiert werden. So soll z. B. die Konzentration des Textilweichmachers nicht viel höher sein als 30 %, wenn man ein klares flüssiges Waschmittel erhalten will.

Die erfindungsgemässen Verbindungen werden in Mengen von 0,005 bis 1 % oder mehr, bezogen auf das Gewicht des flüssigen oder pulverförmigen, fertigen Waschmittels resp. Textilbehandlungsmittels, zugesetzt. Wasch-/Behandlungsflotten, die die angegebenen Mengen der beanspruchten Aufheller enthalten, verleihen beim Waschen von Textilien aus Cellulosefasern, Polyamidfasern, hochveredelten Cellulosefasern, Polyesterfasern, Wolle etc. diesen einen brillanten Aspekt am Tageslicht.

Die Waschbehandlung wird beispielsweise wie folgt durchgeführt : Die angegebenen Textilien werden während 1 bis 30 Minuten bei 20 bis 100 °C in einem Waschbad behandelt, das 0,1 bis 10 g/kg eines aufgebauten, zusammengesetzten Waschmittels und 0,05 bis 1 %, bezogen auf das Waschmittelgewicht, der beanspruchten Aufhellmittel enthält. Das Flottenverhältnis kann 1 : 3 bis 1 : 50 betragen. Nach dem Waschen wird wie üblich gespült und getrocknet.

Unter geeigneten Waschbedingungen können anstelle der amphoteren Carbonsäuren auch deren leicht verseifbare Ester wie z. B. Methyl- oder Aethylester eingesetzt werden. Auf diese Weise erreicht man unter den Bedingungen der Esterhydrolyse die erfindungsgemässen Effekte innerhalb des praktischen Bereiches für das Waschen von Geweben.

In den Beispielen sind Prozente, soweit nicht anders angegeben, immer Gewichtsprozente. Schmelz- und Siedepunkte sind, sofern nicht anders vermerkt, unkorrigiert und oft unscharf, besonders bei den quaternierten Verbindungen.

Beispiel 1 : 5,6 g der Verbindung der Formel

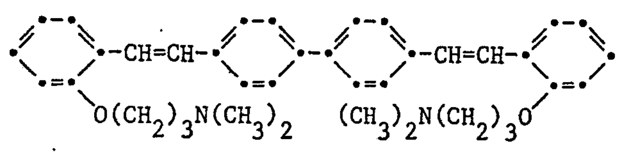

(101)

und 2,6 g Natriumsalz der Chloressigsäure werden in 50 ml Wasser unter Rückfluss verrührt. Durch zeitweiliges Zutropfen von 30 %iger wässriger Natronlauge (ca. 0,1 ml) hält man den pH bei 8-9. Im Verlauf von 3 Stunden tritt vollständige Lösung ein. Danach lässt man auf Raumtemperatur abkühlen, verdünnt mit 50 ml Wasser, nutscht das dick ausgefallene Produkt und wäscht es mit 10 ml Wasser. Nach dem Trocknen im Vakuum bei 100 °C erhält man 6,8 g eines blass-gelben Produktes, das vorwiegend aus der Verbindung der Formel

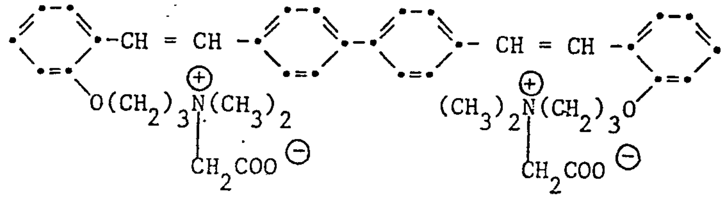

(102)

besteht und noch etwa 4 Mol Kristallwasser enthält. Smp. 225 °C (unscharf), aus Methanol-Aethanol umkristallisiert. Anstelle von Wasser kann auch Dimethylsulfoxid als Lösungsmittel verwendet werden, wobei doppelt so konzentriert gearbeitet werden kann.

Ebenso erhält man das Rohprodukt der Verbindung der Formel (309), das aus Aethanol umkristallisiert wird (Smp. 226 °C) und dasjenige der Formel (215) (Smp. 263 °C).

In analoger Weise erhält man aus 5,6 g der Verbindung der Formel (101) und 6,2 g Natriumsalz der 3-Chlor-2-hydroxy-propansulfosäure in 30 ml Wasser die Verbindung der Formel

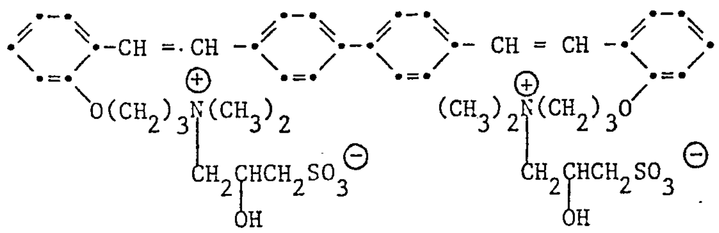

(103)

Smp. 247 °C (unscharf) nach Umkristallisation aus n-Propanol-Wasser 3 : 2.

Beispiel 2 : Eine Lösung von 17,7 g der Verbindung der Formel

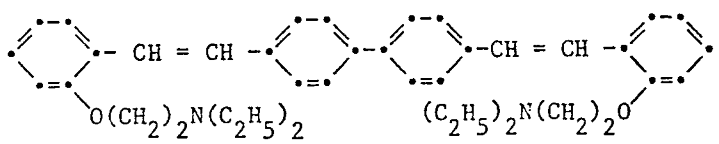

(201)

und 13,4 ml Bromessigsäureäthylester in 120 ml Methyläthylketon wird 2 Stunden bei Rückflusstemperatur verrührt, wobei sich das Reaktionsprodukt nach einiger Zeit allmählich ausscheidet. Darnach ist eine Probe klar löslich in Wasser. Die Mischung wird bei Raumtemperatur abgenutscht, wiederholt mit Methyläthylketon gewaschen und im Vakuum bei 100 °C getrocknet. Man erhält 24,4 g eines hellgelben Produktes der Formel

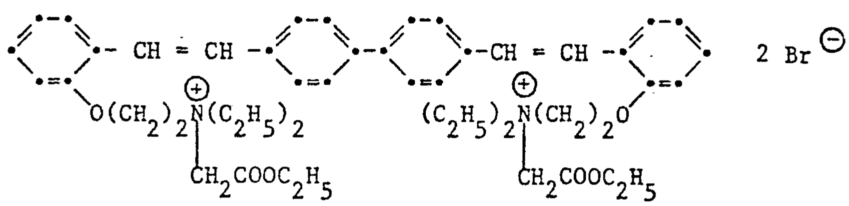

(202)

vom Smp. 159-166 °C (unverändert nach Umkristallisieren aus Isopropanol).

Zu einer Lösung von 9,4 g dieses Produktes in 100 ml Wasser tropft man unter Rühren bei 90 °C 10,25 ml 2n Natronlauge rasch zu. Das dabei ausfallende Produkt geht nach einiger Zeit wieder in Lösung. Nach 1/2 Stunde wird mit etwa 20 ml n-Propanol verdünnt und die Lösung im Vakuum in Rotationsverdampfer vollständig eingedampft. Der Rückstand wird aus Methanol-Aethanol umkristallisiert, mit Aethanol gewaschen und im Vakuum bei 100 °C getrocknet. Man erhält 5,6 g der Verbindung der Formel

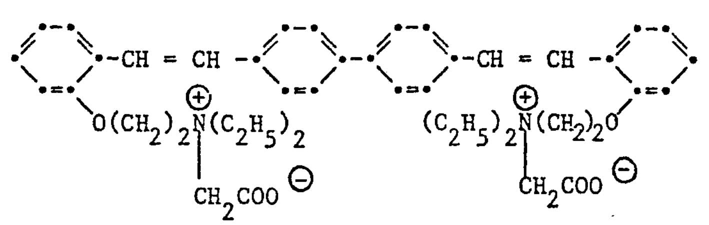

(203)

die noch Kristallwasser und Natriumbromid enthält (Spm. ca. 230 °C, sehr unscharf).

In analoger Weise erhält man die in Tabelle I aufgeführten quaternären Ester und die entsprechenden amphoteren Carbonsäuren.

(Siehe Tabelle 1 Seite 11 ff.)

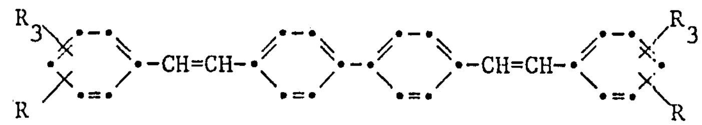

Tabelle I

| Verbindung der Formel | R | $R_3$ | Smp. (°C) | (kristallisierbar aus) |
|---|---|---|---|---|
| (204) | $3-O(CH_2)_2-\overset{\oplus}{N}(C_2H_5)_2$ ; $CH_2COOC_2H_5$ $Br^{\ominus}$ | H | 177° | |
| (205) | $3-O(CH_2)_2-\overset{\oplus}{N}(C_2H_5)_2$ ; $CH_2COO^{\ominus}$ | H | 245° | (Methanol-Aethanol) |
| (206) | $2-O(CH_2)_3-\overset{\oplus}{N}(CH_3)_2$ ; $CH_2COOC_2H_5$ $Br^{\ominus}$ | $3-OCH_3$ | 151° | |
| (207) | $2-O(CH_2)_3-\overset{\oplus}{N}(CH_3)_2$ ; $CH_2COO^{\ominus}$ | $3-OCH_3$ | 272° | (Methanol-Aethanol) |
| (208) | $2-O(CH_2)_3-\overset{\oplus}{N}(CH_3)_2$ ; $CH_2COOC_2H_5$ $Br^{\ominus}$ | 5-Cl | 184° | |
| (209) | $2-O(CH_2)_3-\overset{\oplus}{N}(CH_3)_2$ ; $CH_2COO^{\ominus}$ | 5-Cl | 149° | (n-Propanol-$H_2O$ 8:2) |
| (210) | $2-CH_2-\overset{\oplus}{N}(CH_3)_2$ ; $CH_2COOC_2H_5$ $Br^{\ominus}$ | H | 158° (Zers.) | |

(Fortsetzung)

| Verbindung der Formel | R | $R_3$ | Smp. (°C) | (kristallisierbar aus) |
|---|---|---|---|---|
| (211) | $2\text{-}CH_2\text{-}\overset{\oplus}{N}(CH_3)_2\text{-}CH_2\text{-}COO^{\ominus}$ | H | 173° | (Methanol-Aethanol) |
| (212) | $2\text{-}S(CH_2)_2\text{-}\overset{\oplus}{N}(CH_3)_2\text{-}CH_2COOC_2H_5 \quad Br^{\ominus}$ | H | 274° | |
| (213) | $2\text{-}S(CH_2)_2\text{-}\overset{\oplus}{N}(CH_3)_2\text{-}CH_2COO^{\ominus}$ | H | 237° | (n-Propanol-$H_2O$ 4:1) |
| (214) | $2\text{-}SO_2NH(CH_2)_3\text{-}\overset{\oplus}{N}(CH_3)_2\text{-}CH_2COOC_2H_5 \quad Br^{\ominus}$ | H | 200° (Zers.) | |
| (215) | $2\text{-}SO_2NH(CH_2)_3\overset{\oplus}{N}(CH_3)_2\text{-}CH_2COO^{\ominus}$ | H | 264° (Zers.) | |
| (216) | $2\text{-}COO(CH_2)_2\text{-}\overset{\oplus}{N}(CH_3)_2\text{-}CH_2COOC_2H_5$ | H | 260° | |
| (217) | $2\text{-}COO(CH_2)_2\text{-}\overset{\oplus}{N}(CH_3)_2\text{-}CH_2COO^{\ominus}$ | H | 222° | (n-Propanol-$H_2O$ 7:3) |
| (218) | $2\text{-}CONH(CH_2)_3\text{-}\overset{\oplus}{N}(CH_3)_2\text{-}CH_2COOC_2H_5$ | H | 148° (Zers.) | |
| (219) | $2\text{-}CONH(CH_2)_3\text{-}\overset{\oplus}{N}(CH_3)_2\text{-}CH_2COO^{\ominus}$ | H | 298° (Zers.) | (n-Propanol-$H_2O$ 8:2) |

Das zur Herstellung der Verbindung der Formel (204) benötigte Zwischenprodukt der Formel

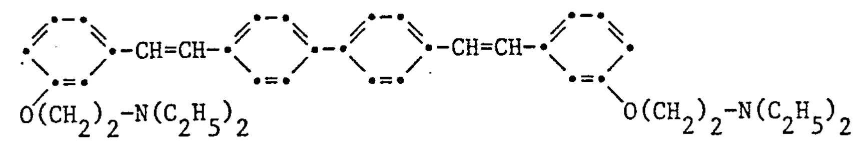

(220)

erhält man beispielsweise wie folgt : Zu einer Lösung von 3,6 g 4,4'-Dimethyl-biphenyl und 12,4 g der Verbindung der Formel

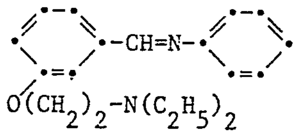

(221)

fügt man 9,0 g Kalium-t-butylat zu. Man rührt die Mischung unter Stickstoff und bringt die Temperatur im Verlauf von etwa 30 Min auf 60 °C, wobei Violettfärbung eintritt. Nach 1-stündigem Nachrühren bei 80 °C lässt man abkühlen und versetzt mit 50 ml Wasser. Das ausgefallene Produkt wird abgenutscht, wiederholt mit Methanol und Wasser neutral gewaschen und im Vakuum bei 100 °C getrocknet. Man erhält 9,0 g der Verbindung der Formel (220), die aus Nonan umkristallisiert wird : Blassgelbe Kristalle, Smp. 174-175 °C.

Zur Herstellung des Anils der Formel (221) erwärmt man 120,3 g 3-Diäthylaminoäthoxybenzaldehyd (Gehalt 92 %) mit 48,9 g Anilin 30 Minuten bei 100 °C und destilliert das entstandene Wasser zuerst bei Normaldruck und dann unter vermindertem Druck ab. Alsdann wird der Rückstand im Hochvakuum fraktioniert destilliert. Man erhält nach Abtrennen eines geringen Vorlaufes 138,0 g eines blass-gelblichen Oels vom Kp. 160-164 °C (0,1 mbar).

In analoger Weise erhält man die entsprechenden Anile der Formel

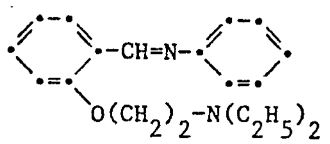

und

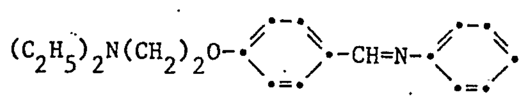

(222) Kp. 160-164° (0,07 mbar) (223) Kp. 170-179° (0,1 mbar)

welche ebenso mit 4,4'-Dimethyl-biphenyl umgesetzt werden. So erhält man beispielsweise die Verbindung der Formel (201) vom Smp. 126-127 °C mit einer Ausbeute von 87 % der Theorie. Anstelle dieser Ausgangsstoffe können auch die entsprechenden o- oder p-Chloranile bzw. p-Xylol eingesetzt werden. Als Katalysatoren eignen sich auch andere Basen wie Kaliumhydroxid oder Natriummethylat.

## Beispiel 3

16,8 g der Verbindung der Formel (101) werden in 30 ml Chloressigsäureäthylester bei 70-100 °C verrührt, bis die Hauptmenge des Reaktionsproduktes ausgefallen ist. Man verdünnt die dick gewordene Suspension mit 70 ml Methyläthylketon und verfährt im weiteren wie in Beispiel 2 beschrieben. Man erhält 23,5 g eines fast farblosen Produktes der Formel

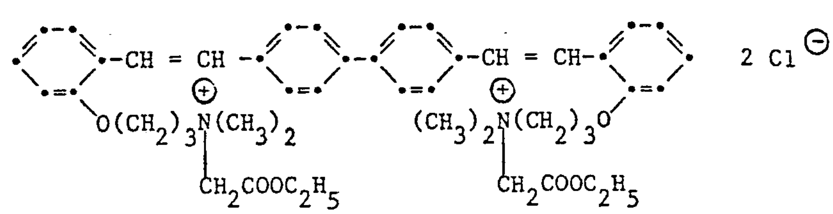

(301)

das aus Aethanol umkristallisiert wird (Smp. 225-230 °C).

12,5 g dieser Verbindung werden gemäss Beispiel 2 mit Natronlauge behandelt und der erhaltene Rückstand aus Aethanol umkristallisiert. Man erhält 8,2 g eines hellgelben Produktes der Formel (102), das noch etwa 6 % Natriumchlorid enthält (Smp. ca. 230 °C, unscharf).

Erhitzt man anstelle der in diesem Beispiel angegebenen Ausgangsprodukte 17,7 g der Verbindung der Formel (201) mit 60 ml 4-Brom-buttersäureäthylester 3 Stunden bei 110 °C und verfährt ansonsten wie vorangehend beschrieben, so erhält man die Verbindung der Formel

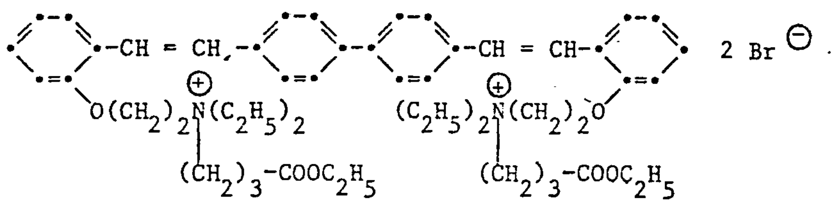
(302)

Smp. 120-140 °C und daraus die Verbindung der Formel

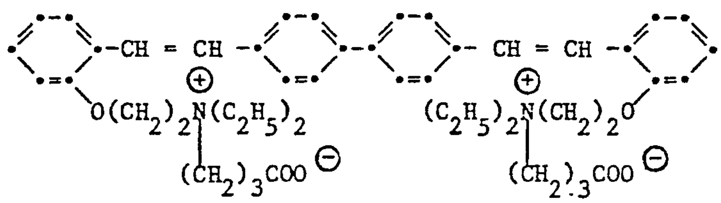
(303)

Smp. 150 °C (unscharf) die noch Natriumbromid enthält.

In analoger Weise erhält man die in Tabelle II aufgeführten Ester und die entsprechenden amphoteren Carbonsäuren.

Tabelle II

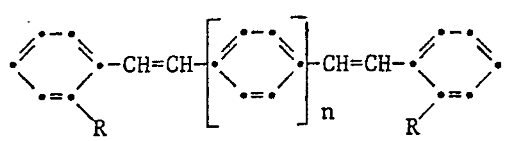

| Verbindung der Formel | n | R | Smp. in °C | (umkristallisiert aus) |
|---|---|---|---|---|
| (304) | 2 | $-O(CH_2)_2-\overset{\oplus}{N}(C_2H_5)_2(CH_2COOC_2H_5)\ Cl^{\ominus}$ | 179° | |
| (305) | 1 | $-O(CH_2)_2-\overset{\oplus}{N}(C_2H_5)_2(CH_2COOC_2H_5)\ Cl^{\ominus}$ | ca 170° (Zers.) | |
| (306) | 1 | $-O(CH_2)_2-\overset{\oplus}{N}(C_2H_5)_2(CH_2COOCH_3)\ Cl^{\ominus}$ | ca. 136° (Zers.) hygroskopisch | |
| (307) | 1 | $-O(CH_2)_2-\overset{\oplus}{N}(C_2H_5)_2(CH_2COO^{\ominus})$ | ca. 230° | (n-Propanol) |
| (308) | 1 | $-O(CH_2)_3-\overset{\oplus}{N}(CH_3)_2(CH_2COOC_2H_5)\ Cl^{\ominus}$ | ca. 220° (Zers.) | |
| (309) | 1 | $-O(CH_2)_3-\overset{\oplus}{N}(CH_3)_2(CH_2COO^{\ominus})$ | ca. 240° | (n-Propanol) |
| (310) | 1 | $-O(CH_2)_2-\overset{\oplus}{N}(C_2H_5)_2((CH_2)_3-COOC_2H_5)\ Br^{\ominus}$ | ca. 152° | (1,2-Dichloräthan) |

(Fortsetzung)

| Verbindung der Formel | n | R | Smp. in°C | (umkristallisiert aus) |
|---|---|---|---|---|
| (311) | 1 | 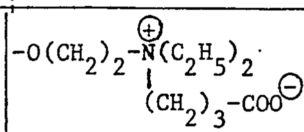 | ca. 203° | (n-Propanol) |
| (312) | 2 | 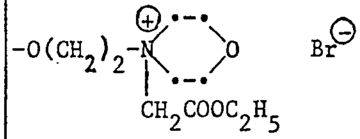 | ca. 200° | |
| (313) | 2 | 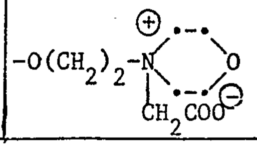 | 267° | (n-Propanol-$H_2O$ 7:3) |

Beispiel 4 : Eine Lösung von 15,4 g der Verbindung der Formel

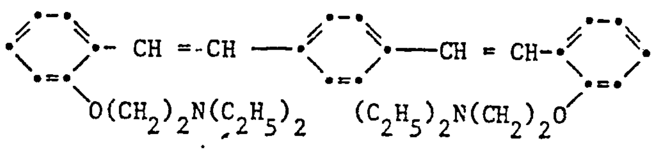

(401)

wird mit 13,4 ml Bromessigsäureäthylester gemäss Beispiel 2 quaterniert. Man erhält 24,2 g der Verbindung der Formel

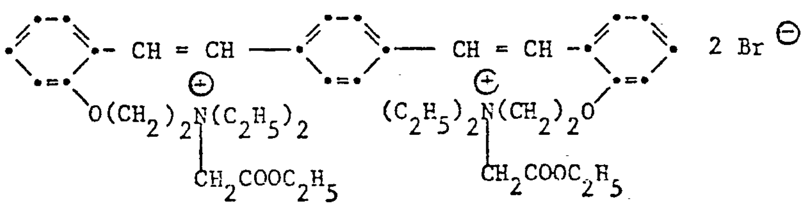

(402)

als hellgelbes Produkt vom Smp. 176-180 °C, das noch 1/2 Mol Kristallwasser enthält.

12,8 g dieser Verbindung werden, wie in Beispiel 2 beschrieben, mit Natronlauge verseift. Der Eindampfrückstand wird mit 200 ml Aceton ausgekocht und die erhaltene Suspension bei Raumtemperatur genutscht. Nach dem Auswaschen des Rückstandes mit Aceton und Trocknen im Vakuum bei 100 °C erhält man 9,7 g der Verbindung der Formel

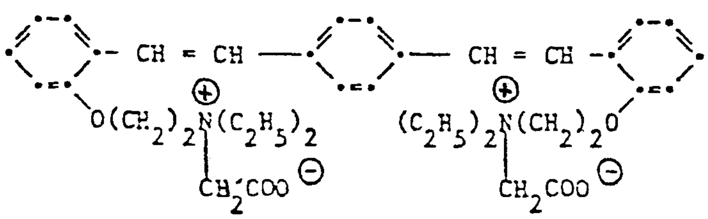

(403)

als hellgelbes Pulver vom Smp. 225 °C (unscharf), das etwa 23 % Natriumbromid enthält.

In analoger Weise erhält man die in Tabelle III aufgeführten quaternierten Ester und die entsprechenden amphoteren Carbonsäuren :

Tabelle III

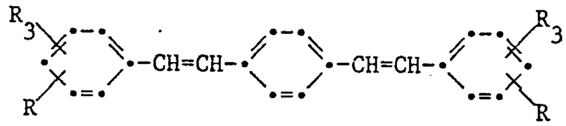

| Verbindung der Formel | R | $R_3$ | Smp. (°C) | (umkristallisiert aus) |
|---|---|---|---|---|
| (404) | 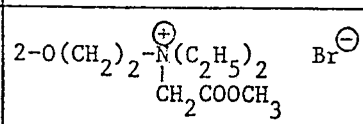 | H | 174° (Zers.) | |
| (405) | 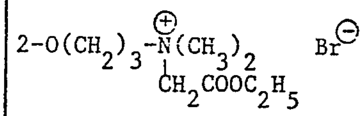 | 5-$CH_3$ | 189° (Zers.) | |
| (406) | 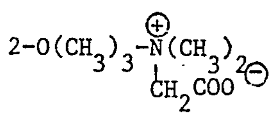 | 5-$CH_3$ | 152° | (n-Propanol) |
| (407) | 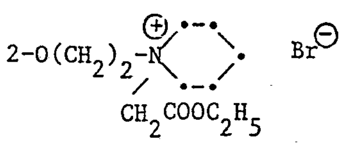 | H | 146° | |
| (408) | 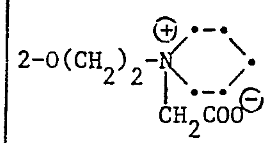 | H | 129° | (Isopropanol) |

Beispiel 5

Zu einer Lösung von 11,2 g der Verbindung der Formel (101) in 50 ml Methyläthylketon tropft man unter Rühren bei Rückflusstemperatur 5,9 g 1,3-Propansulton, wonach sich das Reaktionsprodukt allmählich ausscheidet. Nach Ablauf von 2 Stunden wird abgekühlt, genutscht und der Rückstand wiederholt mit Methyläthylketon gewaschen und im Vakuum bei 100 °C getrocknet. Man erhält 15,5 g eines hellgelben Produktes der Formel

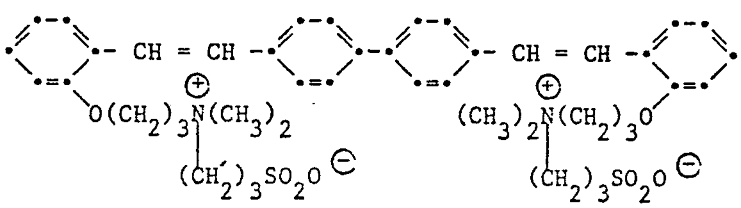

(501)

das 1/2 Mol Kristallwasser enthält. Smp. 298-303 °C (Zers.).

Verwendet man anstelle von 1,3-Propansulton 6,5 g 1,4-Butansulton, so erhält man die Verbindung der Formel

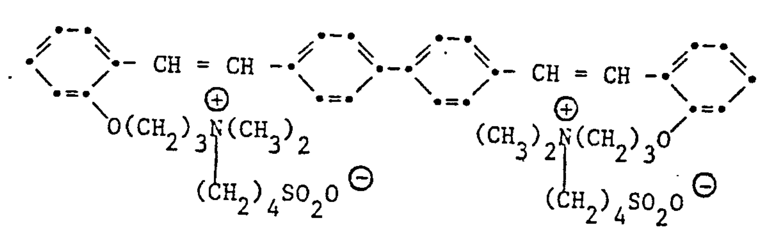

(502)

Smp. 244-248 °C (Zers.), nach Extraktion mit siedendem Wasser, Eindampfen der wässerigen Lösung und Kristallisation des Rückstandes aus Aethanol.

In analoger Weise erhält man die Verbindung der Formel

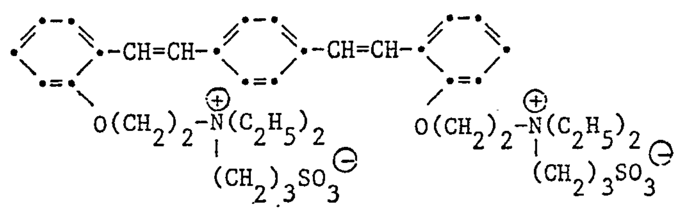

(503)

das 1 Mol Kristallwasser enthält (Smp. 235 °C, unscharf).

Beispiel 6

Zu einer Mischung von 3,8 g 1,4-Bis-(diäthoxyphosphonomethyl)-benzol und 6,2 g der Verbindung der Formel

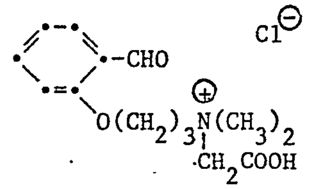

(601)

in 30 ml Dimethylformamid tropft man nach Verdrängen der Luft durch Stickstoff unter Rühren 8,9 g einer 30,5 %igen methanolischen Natriummethylatlösung, so dass die Temperatur nicht über 40 °C ansteigt. Man hält die Temperatur 2 Stunden bei 40-45 °C, neutralisiert mit wässriger Salzsäure auf pH 7 und dampft die Lösung im Vakuum am Rotationsverdampfer vollständig ein. Der Rückstand wird in 40 ml Isopropanol aufgekocht, nach dem Abkühlen im Eisbad filtriert, mit Isopropanol gewaschen und getrocknet. Das erhaltene Produkt wird zur Entfernung der Hauptmenge von Natriumchlorid aus n-Propanol umkristallisiert. Man erhält 2,6 g der Verbindung der Formel (309).

Die Verbindung der Formel (601) kann wie folgt hergestellt werden:

Zu einer Lösung von 26,2 g 2-(3-Dimethylaminopropoxi)-benzaldehyd in 100 ml Aethylmethylketon tropft man bei Rückflusstemperatur 11,7 ml Chloressigsäureäthylester. Nach 1-stündigem Rühren bei Rückflusstemperatur lässt man abkühlen, nutscht das ausgeschiedene Produkt ab, wäscht es wiederholt mit Aethylmethylketon und trocknet es im Vakuum bei 60 °C. Man erhält 25,8 g der Verbindung der Formel

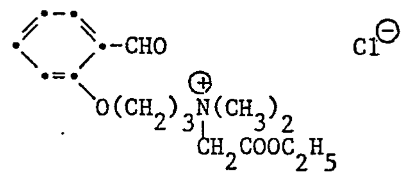

(602)

in Form farbloser Kristalle vom Smp. 190-193 °C.

11,9 g dieses Produktes werden in 12,7 ml Wasser und 7,3 g Salzsäure 36 %ig 1 Stunde bei Rückflusstemperatur verrührt. Die erhaltene Lösung wird im Rotationsverdampfer unter Vakuum vollständig eingedampft und der Rückstand in 50 ml heissem n-Propanol gelöst. Beim Abkühlen fällt das Reaktionsprodukt in farblosen Kristallen aus. Dieses wird bei 0 °C abgenutscht, wiederholt mit n-Propanol gewaschen und im Vakuum bei 100 °C getrocknet. Man erhält 7,9 g der Verbindung (601) vom Smp. 200-203 °C.

Beispiel 7

14,0 g der Verbindung der Formel (101) werden in 15 ml 1,3-Dichlor-2-propanol etwa 3 Stunden bei 90-100 °C verrührt. Man versetzt die erhaltene Lösung mit 250 ml Methyläthylketon, wobei das Reaktionsprodukt ausfällt. Dieses wird bei Raumtemperatur abgenutscht, wiederholt mit Methyläthylketon gewaschen und im Vakuum bei 50 °C getrocknet. Man erhält 18,0 g der Verbindung der Formel

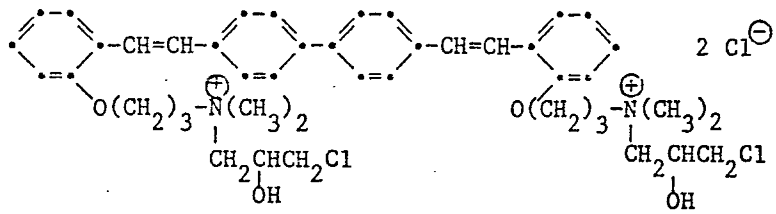

(701)

Smp. ca. 155 °C (Zers.).

8,2 g dieses Produktes und 3,2 g Natriumsulfit 96 %ig werden in 50 ml Wasser und 15 ml n-Propanol über Nacht bei Rückflusstemperatur verrührt. Das nach dem Abkühlen ausgefallene Produkt wird abgenutscht, mit Eiswasser gewaschen und im Vakuum bei 100 °C getrocknet. Man erhält 7,4 g Rohprodukt der Verbindung der Formel (103).

## Beispiel 8

Eine Mischung von 5,1 g der Verbindung der Formel (401), 4 ml Aethanol und 2,35 ml Bromessigsäureäthylester wird unter Rühren auf Rückflusstemperatur (82 °C) erhitzt, wobei Lösung eintritt. Nach Ablauf einer Stunde ist die Quaternierung beendet, d. h. eine Probe ist klar wasserlöslich und zeigt kein Ausgangsprodukt mehr im Dünnschichtchromatogramm. Im Verlauf von etwa 5 Min tropft man nun 10,5 ml 2n Natronlauge hinzu, rührt weiter bei Rückflusstemperatur, bis die Lösung fast neutral geworden ist (ca. 1 Stunde) und lässt abkühlen. Man erhält eine stabile wässrige Lösung der Verbindung der Formel (403) mit einem Gehalt von 27,2 %, die noch etwas Alkohol, Natriumbromid und Glykolsäure (z. B. als Natriumsalz) enthält.

Verwendet man in diesem Beispiel bei sonst gleichem Vorgehen entsprechende Mengen Bromessigsäuremethylester und Methanol, destilliert das Methanol nach Zugabe der Natronlauge ab und stellt mit 0,1 ml konz. Ammoniak auf pH 8, so erhält man eine entsprechende wässrige Lösung der Verbindung der Formel (403).

## Beispiel 9

In ähnlicher Weise, wie in den vorangehenden Beispielen beschrieben, erhält man die in den Tabellen IV und V aufgeführten Verbindungen.

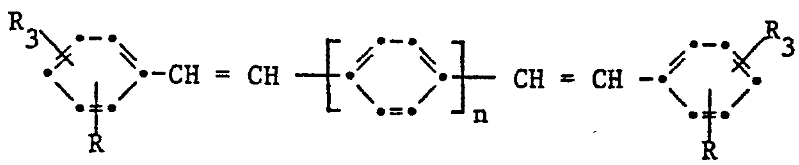

Tabelle IV

| Verb. Nr. | R | $R_3$ | n | Fluoreszenz |
|---|---|---|---|---|
| (901) | 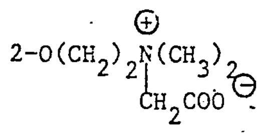 | H | 2 | blau |
| (902) | 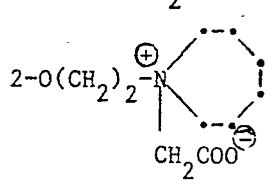 | H | 2 | " |
| (903) | 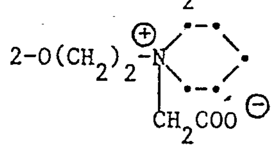 | H | 2 | " |
| (904) | 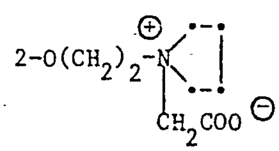 | H | 2 | " |

(Fortsetzung)

| Verb. Nr. | R | $R_3$ | n | Fluoreszenz |
|---|---|---|---|---|
| (905) | 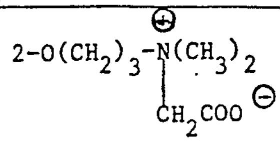 | 5-$CH_3$ | 2 | grünlich-blau |
| (906) | 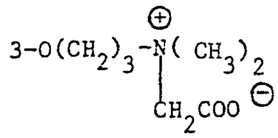 | H | 2 | violett-blau |
| (907) | 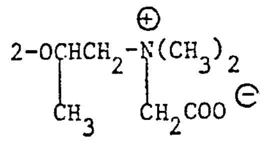 | Mischung | 2 | blau |
| (908) | 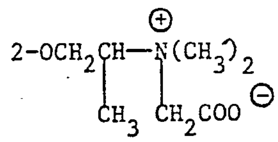 | H | | |
| (909) | 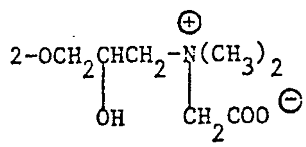 | H | 2 | " |
| (910) | 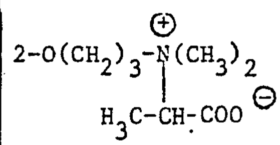 | H | 2 | " |
| (911) | 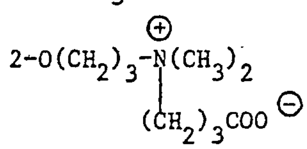 | H | 2 | " |
| (912) | 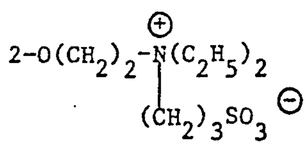 | H | 2 | " |
| (913) | 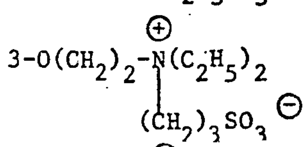 | H | 2 | " |
| (914) | 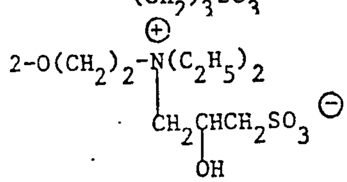 | H | 2 | blau |
| (915) | 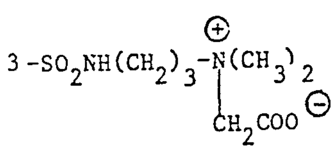 | H | 2 | " |

(Fortsetzung)

| Verb. Nr. | R | $R_3$ | n | Fluoreszenz |
|---|---|---|---|---|
| (916) | 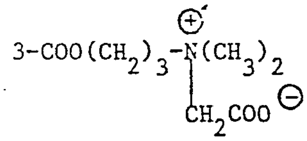 | H | 2 | " |
| (917) | 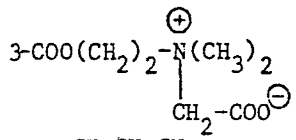 | 6-$CH_3$ | 2 | " |
| (918) | 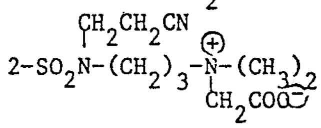 | H | 2 | grünblau |
| (919) | 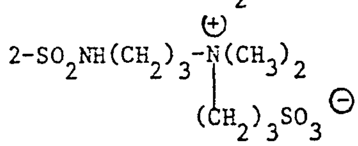 | H | 2 | " |
| (920) | 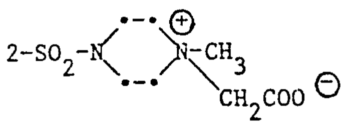 | H | 2 | " |
| (921) | 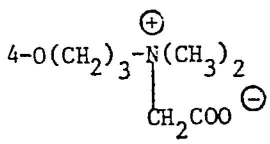 | H | 1 | " |
| (922) | 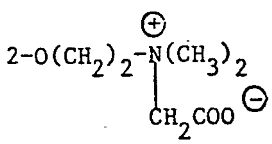 | H | 1 | blau |
| (923) | 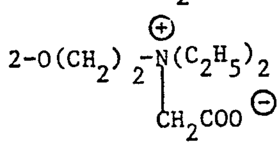 | 5-t-$C_4H_9$ | 1 | grünlich-blau |
| (924) | 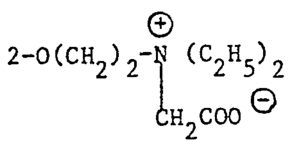 | 3-$CH_3$ | 1 | blau |
| (925) | 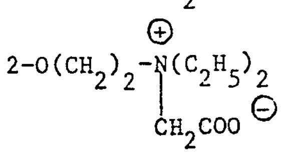 | 4-$CH_3$ | 1 | grünlich-blau |
| (926) | 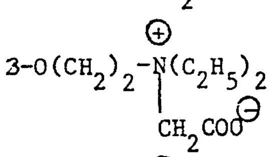 | 2-Cl | 1 | blau |
| (927) | 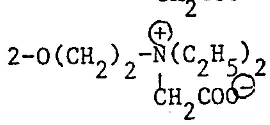 | 3-Cl | 1 | blau |

(Fortsetzung)

| Verb. Nr. | R | $R_3$ | n | Fluoreszenz |
|---|---|---|---|---|
| (928) | 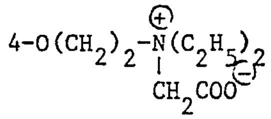 | 2-Cl | 1 | grünlich-blau |
| (929) | 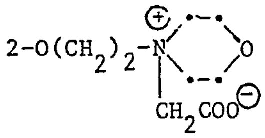 | H | 1 | blau |
| (930) | 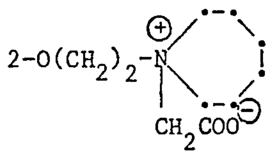 | H | 1 | " |
| (931) | 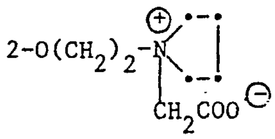 | H | 1 | " |
| (932) | 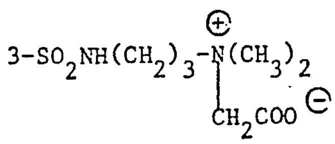 | 4-Cl | 1 | " |
| (933) | 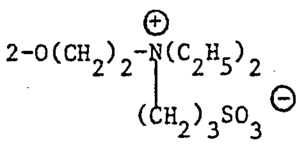 | H | 1 | blau |
| (934) | 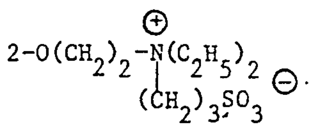 | H | 1 | " |
| (935) | 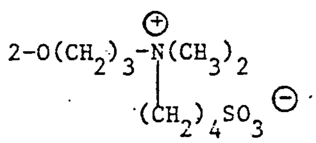 | H | 1 | " |
| (936) | 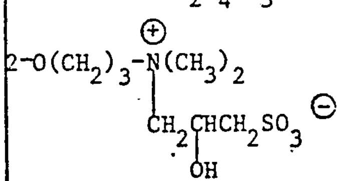 | H | 1 | " |
| (937) | 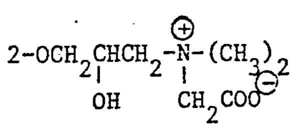 | H | 1 | " |
| (938) | 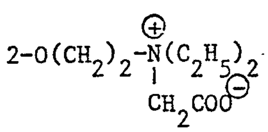 | $3-CH_2CH=CH_2$ | 1 | " |

(Fortsetzung)

| Verb. Nr. | R | $R_3$ | n | Fluoreszenz |
|---|---|---|---|---|
| (939) | $4\text{-}CH_2\overset{\oplus}{N}(CH_3)_2$<br>$CH_2COO^{\ominus}$ | H | 2 | " |
| (940) | $3\text{-}O(CH_2)_2\text{-}\overset{\oplus}{N}(C_2H_5)_2$<br>$CH_2COO^{\ominus}$ | 2-Cl | 1 | " |
| (941) | $3\text{-}COO(CH_2)_2\text{-}\overset{\oplus}{N}(CH_3)_2$<br>$CH_2COO^{\ominus}$ | $6\text{-}CH_3$ | 1 | " |
| (942) | $2\text{-}CONH(CH_2)_3\text{-}\overset{\oplus}{N}(CH_3)_2$<br>$CH_2COO^{\ominus}$ | H | 1 | " |
| (943) | $3\text{-}CONH(CH_2)_3\text{-}\overset{\oplus}{N}(CH_3)_2$<br>$CH_2COO^{\ominus}$ | $6\text{-}CH_3$ | 1 | blau-violett |

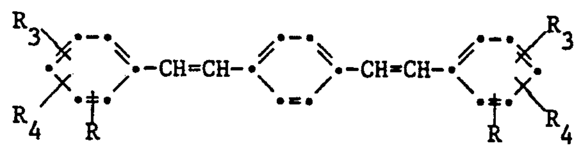

Tabelle V

| Verb. Nr. | R | $R_3$ | $R_4$ | Fluoreszenz |
|---|---|---|---|---|
| (944) | $2\text{-}O(CH_2)_2\text{-}\overset{\oplus}{N}(C_2H_5)_2$<br>$CH_2COO^{\ominus}$ | $3\text{-}CH_3$ | $5\text{-}CH_3$ | blau |
| (945) | $2\text{-}O(CH_2)_2\text{-}\overset{\oplus}{N}(C_2H_5)_2$<br>$CH_2COO^{\ominus}$ | $4,5\text{-}(CH_2)_3$ | | grünlichblau |
| (946) | $2\text{-}O(CH_2)_2\text{-}\overset{\oplus}{N}(C_2H_5)_2$<br>$CH_2COO^{\ominus}$ | $4,5\text{-}(CH_2)_4$ | | " |
| (947) | $2\text{-}O(CH_2)_2\text{-}\overset{\oplus}{N}(C_2H_5)_2$<br>$CH_2COO^{\ominus}$ | 3-Cl | 5-Cl | " |

(Fortsetzung)

| Verb. Nr. | R | $R_3$ | $R_4$ | Fluoreszenz |
|---|---|---|---|---|
| (948) | $4\text{-}O(CH_2)_2\text{-}\overset{\oplus}{N}(C_2H_5)_2$ <br> $\vert$ <br> $CH_2COO^{\ominus}$ | $2\text{-}CH_3$ | $5\text{-}CH(CH_3)_2$ | " |
| (949) | $2\text{-}O(CH_2)_2\text{-}\overset{\oplus}{N}(C_2H_5)_2$ <br> $\vert$ <br> $CH_2COO^{\ominus}$ | $4\text{-}CH_3$ | 5-Cl | " |

Beispiel 10

Ein gebleichtes Baumwollgewebe wird im Flottenverhältnis 1 : 30 während 15 min in einer 40 °C warmen, wässrigen Flotte gewaschen, die pro Liter 0,4 g Schmutz, wie er dem menschlichen Hautfett entpricht (« Spangler Soil »), 1,5 g eines flüssigen Waschmittels a), b), oder c) und 0,1 % eines Aufhellers der Formel (102), (203), (219), (307), (309), (313) oder (403) bezogen auf das Waschmittel, enthält. Anschliessend wird das Baumwollgewebe während 20 Sekunden im fliessenden Trinkwasser gespült und bei 70 °C im Trockenschrank getrocknet. Das so behandelte Gewebe weist einen ebenso hohen Aufhelleffekt auf, wie ein Gewebe, das ohne diese Schmutzzugabe gewaschen wird, auch nach 5- bis 10-fach wiederholtem Waschprozess.

« Spangler Soil » besteht aus einer Mischung verschiedener Fettsäuren, Ester, Fetten Oelen u. a., siehe W. G. Spangler et al., « A laboratory Method for Testing laundry Products for Detergency », J. Am. Oil. Chem. Soc. *42*, 723-727 (1965). Die Einarbeitung erfolgt über das flüssige Waschmittel vor der Verdünnung mit Wasser.

Das verwendete flüssige Waschmittel wird durch Mischen der folgenden Komponenten hergestellt (Gew.-%) :

| | |
|---|---|
| a) Aethoxylierte Alkohole ($C_{11\text{-}12}$-Alkohole mit 5-6 Mol Aethylenoxid | 64 % |
| 1-Methyl-1-oleylamidoäthyl-2-oleylimidazolinium-methosulfat | 20 % |
| Wasser | 13 % |
| Uebliche Zusätze | 3 % |
| b) Aethoxylierte Alkohole ($C_{12\text{-}13}$-Alkohole mit 6,5 Mol Aethylenoxid) | 55 % |
| 1-Methyl-1-talgamidoäthyl-2-talgimidazolinium-methosulfat | 26 % |
| Wasser | 13 % |
| Isopropanol | 5 % |
| Uebliche Zusätze | 1 % |
| c) Aethoxylierte Alkohole ($C_{14\text{-}15}$-Alkohole mit 7 Mol Aethylenoxid) | 12,0 % |
| Aethoxylierte Alkohole ($C_{12\text{-}13}$-Alkohole mit 6,5 Mol Aethylenoxid) | 12,0 % |
| Nicht gehärtetes Di-talg-dimethyl-ammoniumchlorid | 6,4 % |
| Aethanol | 15,0 % |
| Natriumbicarbonat | 0,3 % |
| Uebliche Zusätze | 0,6 % |
| Wasser | 53,7 % |

Beispiel 11

Ein gebleichtes Baumwollgewebe wird mit einer Flotte foulardiert, welche 1 g/l des Aufhellers der Formel (307), (309), (403) oder (408), 1 g/l eines Anlagerungsproduktes von 1 Mol Stearylalkohol an 35 Mol Aethylenoxid, 1 g/l eines Anlagerungsproduktes von 1 Mol p-tert.-Octylphenol an 8 Mol Aethylenoxid, 90 ml Aethanol (95 %) und 2 g/l Natriumtripolyphosphat enthält. Das Gewebe wird bis zu einer Flottenaufnahme von 75 % abgequetscht. Das so behandelte Gewebe wird nun in einen Färbeapparat gegeben,

der so viel Wasser enthält, dass das Flottenverhältnis 1 : 25 beträgt. Die Applikation erfolgt nun nach folgendem Temperaturprogramm :

30-70 °C/10 Minuten

70 °C/20 Minuten.

Danach wird das Gewebe in kaltem enthärtetem Wasser gespült, zentrifugiert und mit einem 155 °C warmen Bügeleisen getrocknet. Das so behandelte Baumwoll-Gewebe weist einen guten Aufhelleffekt auf.

Beispiel 12

Ein Polyacrylnitril-Gewebe (Orlon 75) wird auf einem Färbeapparat bei einem Flottenverhältnis von 1 : 20 mit einem wässrigen Bad, das

0,1 % des Aufhellers der Formel (209), (307), (309), (313), (406), oder (408) bezogen auf das Warengewicht,

1 g/l eines Anlagerungsproduktes von 35 Mol Aethylenoxid an 1 Mol Stearylalkohol und

1,5 ml/l Ameisensäure 85 %

enthält, behandelt.

Die Applikation erfolgt gemäss folgendem Temperaturprogramm :

40-97 °C/30 Minuten

97 °C/30 Minuten

97-40 °C/15 Minuten.

Anschliessend wird das Polyacrylnitril-Gewebe während 20 Sekunden in fliessendem enthärtetem Wasser gespült und bei 70 °C im Trockenschrank getrocknet. Das so behandelte Gewebe weist einen guten Aufhelleffekt auf.

Beispiel 13

Ein gebleichtes Baumwoll-Gewebe wird im Flottenverhältnis 1 : 20 während 15 Min in einer 30 °C warmen, wässrigen Weichspülerflotte behandelt, die pro Liter

0,2 g quaternäres Dimethyldistearylammoniumchlorid und

0,01 g des Aufhellers der Formel (102), (203), (219), (307), (309), (313) oder (403)

enthält.

Anschliessend wird das Baumwoll-Gewebe während 5 Sekunden in fliessendem Trinkwasser gespült und bei 70 °C im Trockenschrank getrocknet. Das so behandelte Baumwoll-Gewebe weist einen guten Aufhelleffekt auf.

Beispiel 14

Ein gebleichtes Baumwoll-Gewebe wird im Flottenverhältnis 1 : 20 während 15 Minuten in einer 40 °C warmen, wässrigen Flotte gewaschen, die pro Liter

0,5 g eines Anlagerungsproduktes von 10 Mol Aethylenoxid an einem Mol Stearylalkohol und

0,01 g des Aufhellers der Formel (102), (203), (219), (307), (309), (313) oder (403)

enthält.

Anschliessend wird das Baumwoll-Gewebe während 20 Sekunden in fliessendem Trinkwasser gespült und bei 70 °C im Trockenschrank getrocknet. Das so behandelte Baumwoll-Gewebe weist einen guten Aufhelleffekt auf.

Fügt man zur vorangehend beschriebenen Flotte noch 0,2 g/l Aktivchlor in Form von Natriumhypochlorit hinzu und verfährt wie beschrieben, so erhält man ebenso gute Aufhelleffekte.

Beispiel 15

0,3 g des Aufhellers der Formel (102), (207), (209), (211), (213), (313) oder (406) werden in 270 ml enthärtetem Wasser gelöst, die 0,27 g Essigsäure (80 %) enthalten. Dieser Lösung werden 0,3 g des Anlagerungsproduktes von 35 Mol Aethylenoxid an 1 Mol Stearylalkohol, 0,3 g des Anlagerungsproduktes von 8-9 Mol Aethylenoxid an 1 Mol p-tert. Octylphenol und 30 ml Aethanol (95 %) zugegeben. Zu dieser auf 50 °C erwärmten Aufhellerlösung gibt man ein 9 g schweres Polyamidgewebe. Innerhalb von 10 Minuten steigert man die Temperatur auf 100 °C, belässt bei dieser Temperatur 20 Minuten und kühlt anschliessend innerhalb 5 Minuten auf 50 °C ab. Alsdann wird das Gewebe in enthärtetem Wasser gespült, zentrifugiert und mit einem Bügeleisen bei 180 °C getrocknet. Das so behandelte Gewebe weist einen guten Aufhelleffekt auf.

Beispiel 16

5 g Faserstoff (bestehend aus gebleichter Sulfitcellulose und gebleichter Buchencellulose 1 : 1) in 150 ml Wasser, enthaltend 5 mg eines kationaktiven Polyätheramins werden in einem Mixer mit 50 ml

Aufhellerlösung, enthaltend 4 mg entsprechend einer Konzentration von 0,08 % des Aufhellers der Formel (102), (203), (307) oder (309) während 15 Minuten gemischt. Anschliessend werden 1,5 Gew.% Leim, z. B. Bewoidleim®, 2,5 Gew.% Aluminiumsulfat und 0,1 % eines kationaktiven Polyätheramins (bezogen auf das Fasertrockengewicht) zugegeben und mit Wasser von ca. 10° dH auf 1 000 ml verdünnt. Mit dieser Fasersuspension wird ein Papierblatt gebildet, das einen guten Aufhelleffekt aufweist.

Beispiel 17

5 g Faserstoff (bestehend aus gebleichter Sulfitcellulose und gebleichter Buchencellulose 1 : 1) in 150 ml Wasser, enthaltend 5 mg eines Polyäthylenimins, werden in einem Mixer mit 50 ml Aufhellerlösung, enthaltend 4 mg entsprechend einer Konzentration von 0,08 % des Aufhellers der Formel (102), (203), (307) oder (309) während 15 Minuten gemischt. Anschliessend werden 1,5 Gew.% Leim, z. B. Bewoidleim® 2,5 Gew.% Aluminiumsulfat und 0,1 % eines Polyäthylenimins (bezogen auf das Fasertrockengewicht) zugegeben und mit Wasser von ca. 10° dH auf 1 000 ml verdünnt. Mit dieser Fasersuspension wird ein Papierblatt gebildet, das einen guten Aufhelleffekt aufweist.

Beispiel 18

Ein Gewebe aus gebleichter Wolle wird mit einer Flotte foulardiert, welche 0,05 % des Aufhellers der Formel (102), (403), (406) oder (501), bezogen auf das Gewicht des Gewebes, 1 g/l des Anlagerungsproduktes von 35 Mol Aethylenoxid an 1 Mol Stearylalkohol, 1 g/l des Anlagerungsproduktes von 8-9 Mol Aethylenoxid an 1 Mol 4-Isooctylphenol und 1 ml/l Essigsäure (80 %) enthält. Das Gewebe wird bis zu einer Flottenaufnahme von 70 % abgequetscht. Das so behandelte Gewebe wird nun in einen Färbeapparat gegeben, der so viel Wasser enthält, dass das Flottenverhältnis 1 : 25 beträgt. Die Applikation erfolgt nun nach folgendem Temperaturprogramm :

30-60 °C/10 Minuten
60 °C/75 Minuten.

Danach wird das Gewebe in kaltem enthärtetem Wasser gespült, zentrifugiert und mit einem 155 °C warmen Bügeleisen getrocknet. Das so behandelte Wollgewebe weist einen guten Aufhelleffekt auf.

**Ansprüche** (für die Vertragsstaaten : BE, CH, DE, FR, GB, IT, LI, NL, SE)

1. Amphotere Styrolderivate der Formel

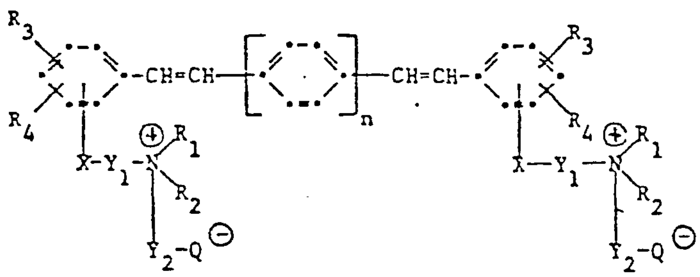

(1)

worin

X Sauerstoff, Schwefel, die direkte Bindung, $-SO_2N(R_5)-$, $-CON(R_5)-$ oder $-COO-$,
$Y_1$ und $Y_2$ unabhängig voneinander $C_1$-$C_4$-Alkylen oder Hydroxypropylen,
$R_1$ und $R_2$ unabhängig voneinander $C_1$-$C_4$-Alkyl oder zusammen mit dem N-Atom einen Pyrrolidin-, Piperidin-, Hexamethylenimin- oder Morpholinring und $R_1$ zusammen mit $R_5$ auch einen Piperazinring,
$R_3$ und $R_4$ Wasserstoff-, $C_{1-4}$-Alkyl, Chlor, $C_{1-4}$-Alkoxy, $C_{3-4}$-Alkenyl oder in o-Stellung zueinander zusammen eine Trimethylen- oder Tetramethylengruppe,
$R_5$ Wasserstoff, $C_{1-4}$-Alkyl, Cyanoäthyl oder zusammen mit $R_1$ einen Piperazinring,
Q $-COO$ oder $-SO_3$ und
n die Zahl 1 oder 2

bedeuten.

2. Amphotere Styrolderivate gemäss Anspruch 1 der Formel

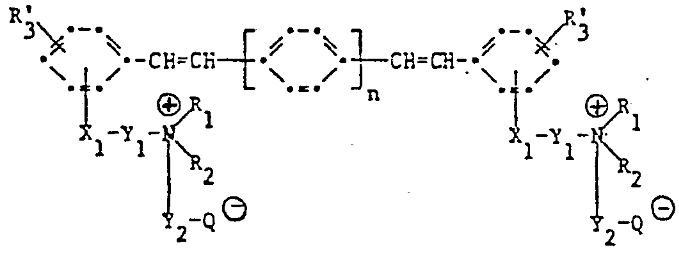

(2)

worin

$R_1$, $R_2$, $Y_1$, $Y_2$, Q und n die in Anspruch 1 angegebene Bedeutung haben und
$X_1$ Sauerstoff, die direkte Bindung, —CONH— oder —COO— und
$R'_3$ Wasserstoff, $C_{1-4}$-Alkyl, Methoxy oder Chlor bedeuten.

3. Amphotere Styrolderivate gemäss Anspruch 2 der Formel

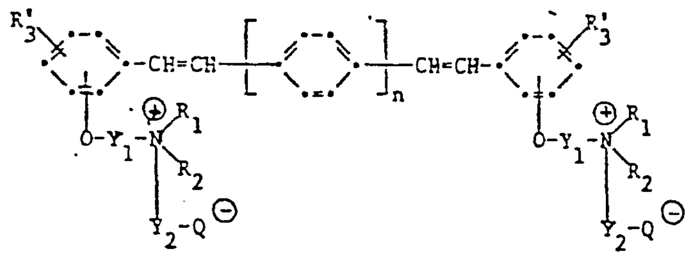

(3)

worin $R_1$, $R_2$, $R'_3$, $Y_1$, $Y_2$, Q und n die in Anspruch 2 angegebene Bedeutung haben.

4. Amphotere Styrolderivate gemäss Anspruch 3 der Formel

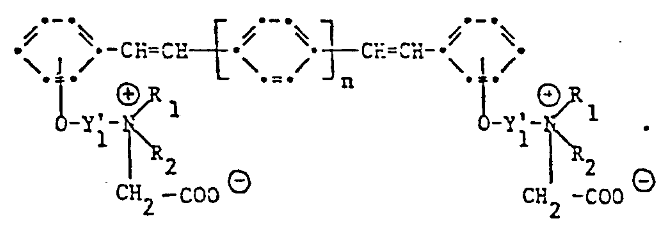

(4)

worin

$Y'_1$ $C_1$-$_4$-Alkylen bedeutet und
$R_1$, $R_2$ und n die in Anspruch 3 angegebene Bedeutung haben.

5. Amphoteres Styrolderivat gemäss Anspruch 4 der Formel

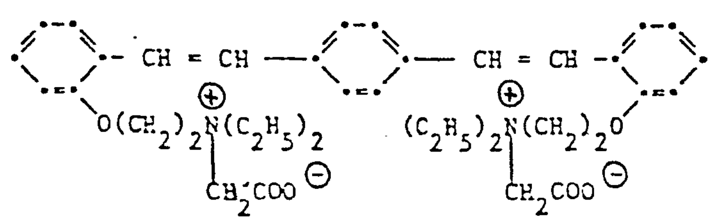

(403)

6. Verfahren zur Herstellung von amphoteren Styrolderivaten der Formel

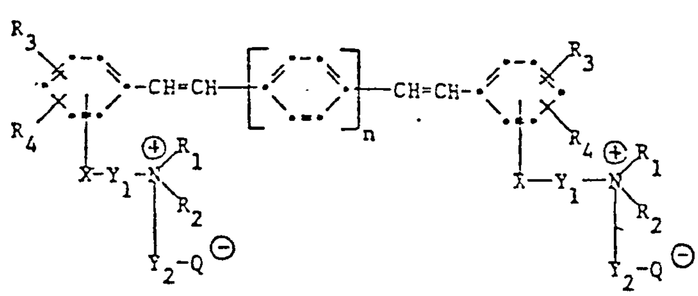

(1)

worin

X Sauerstoff, Schwefel, die direkte Bindung, —$SO_2N(R_5)$—, —$CON(R_5)$— oder —COO—,
$Y_1$ und $Y_2$ unabhängig voneinander $C_1$-$C_4$-Alkylen oder Hydroxypropylen
$R_1$ und $R_2$ unabhängig voneinander $C_1$-$C_4$-Alkyl oder zusammen mit dem N-Atom einen Pyrrolidin-, Piperidin-, Hexamethylenimin- oder Morpholinring und $R_1$ zusammen mit $R_5$ auch einen Piperazinring,
$R_3$ und $R_4$ Wasserstoff-, $C_{1-4}$-Alkyl, Chlor, $C_{1-4}$-Alkoxy, $C_{3-4}$-Alkenyl oder in o-Stellung zueinander zusammen eine Trimethylen- oder Tetramethylengruppe,
$R_5$ Wasserstoff, $C_{1-4}$-Alkyl, Cyanoäthyl oder zusammen mit $R_1$ einen Piperazinring,
Q —COO oder —$SO_3$ und
n die Zahl 1 oder 1

bedeuten, dadurch gekennzeichnet, dass man ein Styrolderivat der Formel

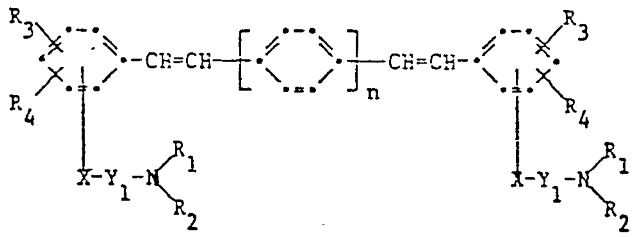

(5)

worin $R_1$, $R_2$, $R_3$, $R_4$, X, $Y_1$ und n die oben angegebene Bedeutung haben, mit einem Halogenid der Formel

$$Hal—Y_2—QH$$

worin $Y_2$ und Q die oben angegebene Bedeutung haben und Hal für Chlor oder Brom stehen oder wenn Q = —COO, mit dessen Salzen, Estern, Amiden, Nitrilen oder Lactonen oder wenn Q = $—SO_3$ mit dessen Salzen, Arylestern oder Sultonen oder wenn $Y_2$ = Hydroxypropylen auch mit den entsprechenden Epoxiden umsetzt und allfällig sich gebildete quaternäre Ammoniumhalogenide verseift.

7. Verwendung von amphoteren Styrolderivaten der in Anspruch 1 definierten Formel (1) zum optischen Aufhellen von organischen Materialien.

8. Verfahren zum optischen Aufhellen von organischen Materialien, dadurch gekennzeichnet, dass man amphotere Styrolderivate der im Anspruch 1 definierten Formel (1) diesen Materialien einverleibt oder auf deren Oberfläche aufbringt.

9. Verfahren gemäss Anspruch 8 zum optischen Aufhellen von Cellulose oder Polyacrylnitril als organisches Material.

10. Waschmittel enthaltend ein oder mehrere amphotere Styrolderivate der im Anspruch 1 definierten Formel (1).

11. Waschmittel gemäss Anspruch 10, welches 10 bis 70 Gew.-% eines nicht-ionogenen Tensides und 1 bis 30 % eines kationischen Textilweichmachers enthält.

12 Waschmittel gemäss Anspruch 11, welches 10 bis 70 Gew.-% eines nicht-ionogenen Tensides und 1 bis 30 Gew.-% eines quartären Derivates des Ammoniaks und/oder des Imidazolins mit je 2 langkettigen aliphatischen Resten als kationischen Textilweichmacher und zur Verleihung der flüssigen Form ein Lösungsmittel enthält.

13. Textilbehandlungsmittel, enthaltend ein oder mehrere amphotere Styrolderivate der im Anspruch 1 definierten Formel (1).

14. Wäschenachbehandlungsmittel, enthaltend ein oder mehrere amphotere Styrolderivate der im Anspruch 1 definierten Formel (1) und einen kationischen Textilweichmacher.

**Ansprüche** (für den Vertragsstaat AT)

1. Waschmittel enthaltend ein amphoteres Styrolderivat der Formel

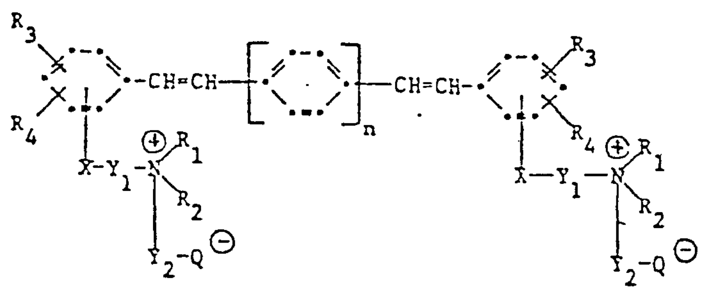

worin

X Sauerstoff, Schwefel, die direkte Bindung, $—SO_2N(R_5)—$, $—CON(R_5)—$ oder —COO—,

$Y_1$ und $Y_2$ unabhängig voneinander $C_1$-$C_4$-Alkylen oder Hydroxypropylen

$R_1$ und $R_2$ unabhängig voneinander $C_1$-$C_4$-Alkyl oder zusammen mit dem N-Atom einen Pyrrolidin-, Piperidin-, Hexamethylenimin- oder Morpholinring und $R_1$ zusammen mit $R_5$ auch einen Piperazinring,

$R_3$ und $R_4$ Wasserstoff-, $C_{1-4}$-Alkyl, Chlor, $C_{1-4}$-Alkoxy, $C_{3-4}$-Alkenyl oder in o-Stellung zueinander zusammen eine Trimethylen- oder Tetramethylengruppe,

$R_5$ Wasserstoff, $C_{1-4}$-Alkyl, Cyanoäthyl oder zusammen mit $R_1$ einen Piperazinring,

Q —COO oder $—SO_3$ und

n die Zahl 1 oder 2

bedeuten.

2. Waschmittel nach Anspruch 1 enthaltend ein amphoteres Styrolderivat der Formel

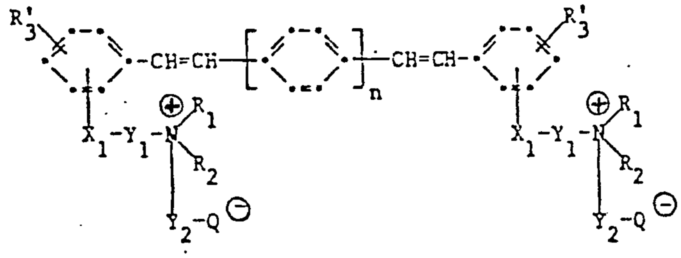

worin

$R_1$, $R_2$, $Y_1$, $Y_2$, Q und n die in Anspruch 1 angegebene Bedeutung haben und

$X_1$ Sauerstoff, die direkte Bindung, —CONH— oder —COO— und

$R_3'$ Wasserstoff, $C_{1-4}$-Alkyl, Methoxy oder Chlor bedeuten.

3. Waschmittel nach Anspruch 2 enthaltend ein amphoteres Styrolderivat der Formel

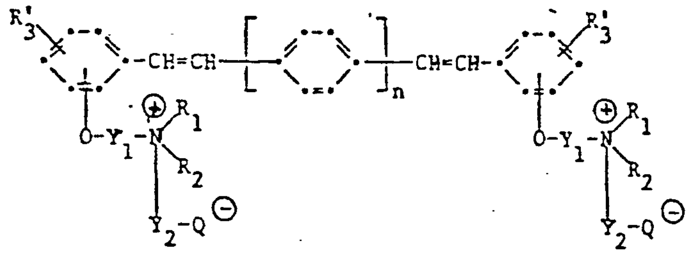

worin $R_1$, $R_2$, $R_3'$, $Y_1$, $Y_2$, Q und n die in Anspruch 2 angegebene Bedeutung haben.

4. Waschmittel nach Anspruch 3 enthaltend ein amphoteres Styrolderivat der Formel

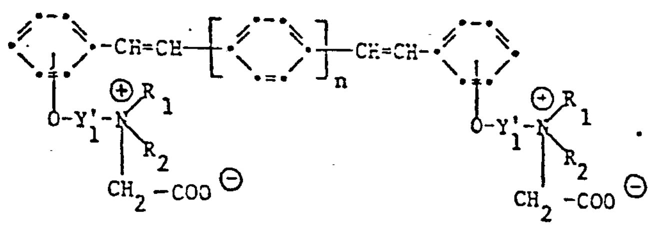

worin

$Y_1'$ $C_{1-4}$-Alkylen bedeutet und

$R_1$, $R_2$ und n die in Anspruch 3 angegebene Bedeutung haben.

5. Waschmittel nach Anspruch 4 enthaltend das amphotere Styrolderivat der Formel

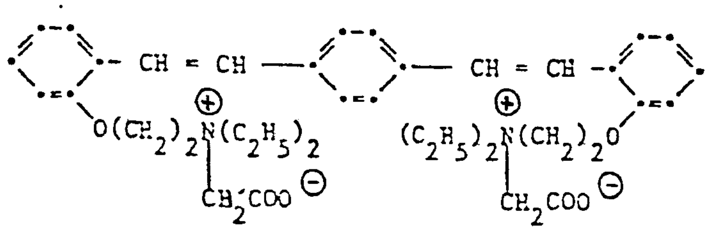

6. Waschmittel nach Anspruch 1, welches noch 10 bis 70 Gew.% eines nicht-ionogenen Tensides und 1 bis 30 % eines kationischen Textilweichmachers enthält.

7. Waschmittel nach Anspruch 6, welches 10 bis 70 Gew.% eines nicht-ionogenen Tensides und 1 bis 30 Gew.% eines quartären Derivates des Ammoniaks und/oder des Imidazolins mit je 2 langkettigen aliphatischen Resten als kationischen Textilweichmacher und zur Verleihung der flüssigen Form ein Lösungsmittel enthält.

8. Verwendung von einem oder mehreren der in Anspruch 1 definierten amphoteren Styrolderivaten der Formel (1) zum optischen Aufhellen von organischen Materialien.

9. Verwendung gemäss Anspruch 8 zum optischen Aufhellen von Cellulose oder Polyacrylnitril als organisches Material.

10. Verfahren zur Herstellung von amphoteren Styrolderivaten der Formel

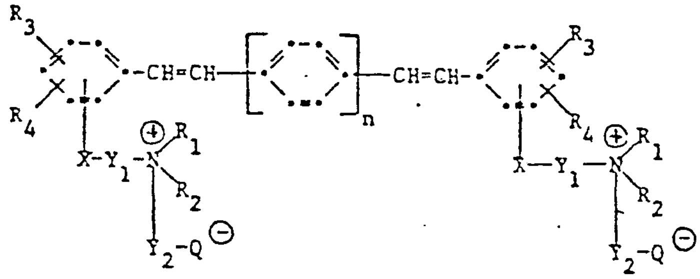

worin

X Sauerstoff, Schwefel, die direkte Bindung, $—SO_2N(R_5)—$, $—CON(R_5)—$ oder $—COO—$,

$Y_1$ und $Y_2$ unabhängig voneinander $C_1$-$C_4$-Alkylen oder Hydroxypropylen

$R_1$ und $R_2$ unabhängig voneinander $C_1$-$C_4$-Alkyl oder zusammen mit dem N-Atom einen Pyrrolidin-, Piperidin-, Hexamethylenimin- oder Morpholinring und $R_1$ zusammen mit $R_5$ auch einen Piperazinring,

$R_3$ und $R_4$ Wasserstoff-, $C_{1-4}$-Alkyl, Chlor, $C_{1-4}$-Alkoxy, $C_{3-4}$-Alkenyl oder in o-Stellung zueinander zusammen eine Trimethylen- oder Tetramethylengruppe,

$R_5$ Wasserstoff, $C_{1-4}$-Alkyl, Cyanoäthyl oder zusammen mit $R_1$ einen Piperazinring,

Q $—COO$ oder $—SO_3$ und

n die Zahl 1 oder 2

bedeuten, dadurch gekennzeichnet, dass man ein Styrolderivat der Formel

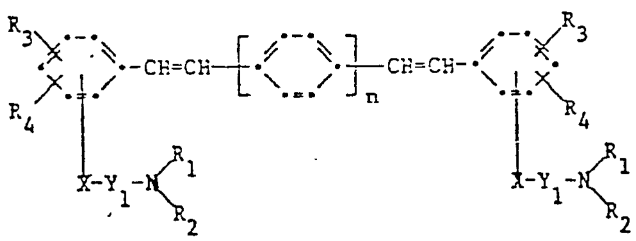

worin $R_1$, $R_2$, $R_3$, $R_4$, X, $Y_1$ und n die oben angegebene Bedeutung haben, mit einem Halogenid der Formel

$$Hal—Y_2—QH$$

worin $Y_2$ und Q die oben angegebene Bedeutung haben und Hal für Chlor oder Brom stehen oder wenn $Q = —COO$, mit dessen Salzen, Estern, Amiden, Nitrilen oder Lactonen oder wenn $Q = —SO_3$ mit dessen Salzen, Arylestern oder Sultonen oder wenn $Y_2$ = Hydroxypropylen auch mit den entsprechenden Epoxiden umsetzt und allfällig sich gebildete quaternäre Ammoniumhalogenide verseift.

**Claims** (for the Contracting States : BE, CH, DE, FR, GB, IT, LI, NL, SE)

1. An amphoteric styrene derivative of the formula

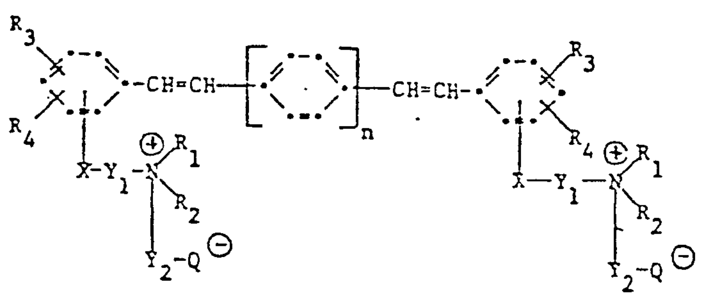

(1)

wherein

X is oxygen, sulfur, a direct bond, $—SO_2N(R_5)—$, $—CON(R_5)—$ or $—COO—$,

$Y_1$ and $Y_2$ are each independently $C_1$-$C_4$ alkylene or hydroxypropylene,

$R_1$ and $R_2$ are each independently $C_1$-$C_4$ alkyl or, together with the N atom, are a pyrrolidine, piperidine, hexamethylene-imine or morpholine ring, and $R_1$ together with $R_5$ is also a piperazine ring,

$R_3$ and $R_4$ are hydrogen, $C_{1-4}$ alkyl, chlorine, $C_{1-4}$ alkoxy or $C_{3-4}$ alkenyl or together, in the o-position relative to each other, are a trimethylene or tetramethylene group,

$R_5$ is hydrogen, $C_{1-4}$ alkyl or cyanoethyl or, together with $R_1$, is a piperazine ring,
Q is —COO or —$SO_3$, and
n is 1 or 2.

2. An amphoteric styrene derivative according to claim 1, of the formula

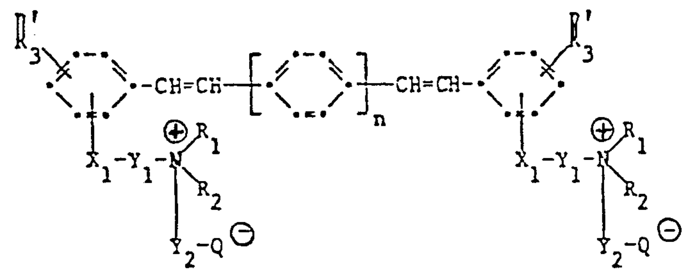

(2)

wherein
$R_1$, $R_2$, $Y_1$, $Y_2$, Q and n are as defined in claim 1,
$X_1$ is oxygen, a direct bond, —CONH— or —COO— and
$R_3'$ is hydrogen, $C_{1-4}$ alkyl, methoxy or chlorine.

3. An amphoteric styrene derivative according to claim 2, of the formula

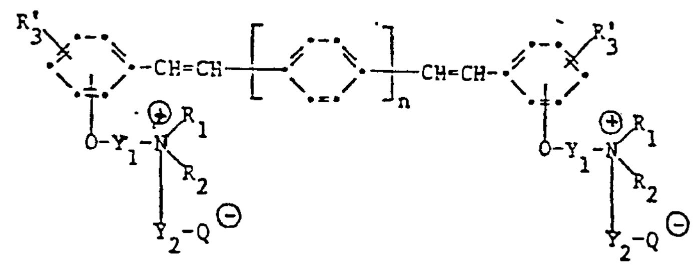

(3)

wherein $R_1$, $R_2$, $R_3'$, $Y_1$, $Y_2$, Q and n are as defined in claim 2.

4. An amphoteric styrene derivative according to claim 3, of the formula

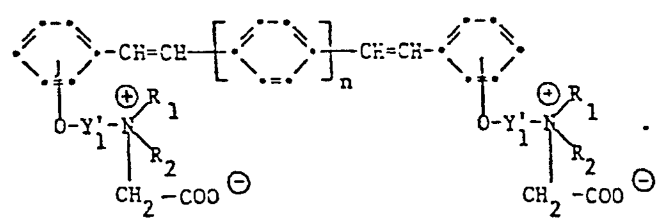

(4)

wherein
$Y_1'$ is $C_{1-4}$ alkylene and
$R_1$, $R_2$ and n are as defined in claim 3.

5. An amphoteric styrene derivative according to claim 4, of the formula

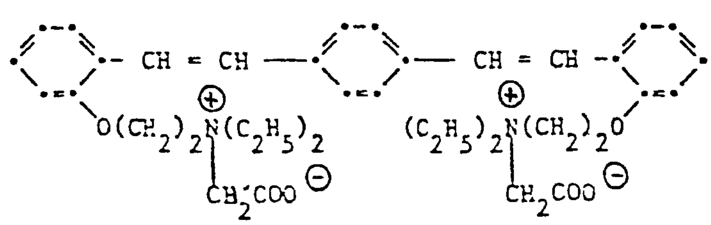

(403)

6. A process for the preparation of an amphoteric styrene derivative of the formula

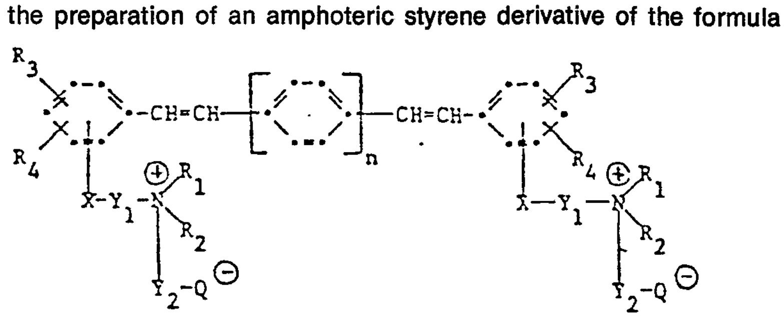

(1)

wherein

X is oxygen, sulfur, a direct bond, —$SO_2N(R_5)$—, —$CON(R_5)$— or —COO—,

$Y_1$ and $Y_2$ are each independently $C_1$-$C_4$ alkylene or hydroxypropylene,

$R_1$ and $R_2$ are each independently $C_1$-$C_4$ alkyl or, together with the N atom, are a pyrrolidine, piperidine, hexamethylene-imine or morpholine ring, and $R_1$ together with $R_5$ is also a piperazine ring,

$R_3$ and $R_4$ are hydrogen, $C_{1-4}$ alkyl, chlorine, $C_{1-4}$ alkoxy or $C_{3-4}$ alkenyl or together, in the o-position relative to each other, are a trimethylene or tetramethylene group,

$R_5$ is hydrogen, $C_{1-4}$ alkyl or cyanoethyl or, together with $R_1$, is a piperazine ring,

Q is —COO or —$SO_3$, and

n is 1 or 2,

which process comprises reacting a styrene derivative of the formula

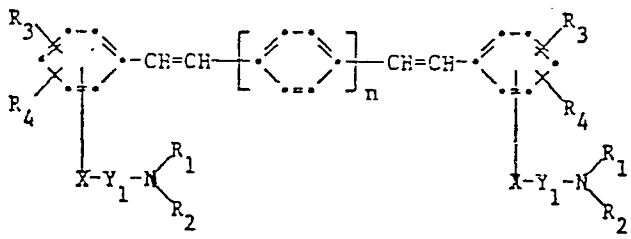

(5)

wherein $R_1$, $R_2$, $R_3$, $R_4$, X, $Y_1$ and n are as defined above, with a halide of the formula

$$Hal—Y_2—QH$$

in which $Y_2$ and Q are as defined above and Hal is chlorine or bromine, or, if Q = —COO, with a salt, ester, amide, nitrile or lactone thereof, or, if $Y_2$ = hydroxypropylene, also with a corresponding epoxide, and saponifying, any quaternary ammonium halide formed.

7. Use of an amphoteric styrene derivative of the formula (1) defined in claim 1, for whitening organic materials.

8. A process for whitening organic materials, which comprises incorporating an amphoteric styrene derivative of the formula (1) as defined in claim 1 into said materials or applying it to the surface thereof.

9. A process according to claim 1 wherein the organic material to be whitened is cellulose or polyacrylonitrile.

10. A detergent composition containing one or more amphoteric styrene derivatives of the formula (1) as defined in claim 1.

11. A detergent composition according to claim 10, which contains 10 to 70 % by weight of a non-ionic surfactant and 1 to 30 % of a cationic textile softener.

12. A detergent composition according to claim 11, which contains 10 to 70 % by weight of a non-ionic surfactant and 1 to 30 % by weight of a quaternary derivative of ammonia and/or of imidazoline, having in each case 2 long-chain aliphatic radicals, as the cationic textile softener, and a solvent to bring the detergent into the liquid form.

13. A fabric conditioner containing one or more amphoteric styrene derivatives of the formula (1) as defined in claim 1.

14. A laundry after-treatment composition containing one or more amphoteric styrene derivatives of the formula (1) as defined in claim 1 and a cationic textile softener.

**Claims** (for the Contracting State AT)

1. A detergent composition which contains an amphoteric styrene derivative of the formula

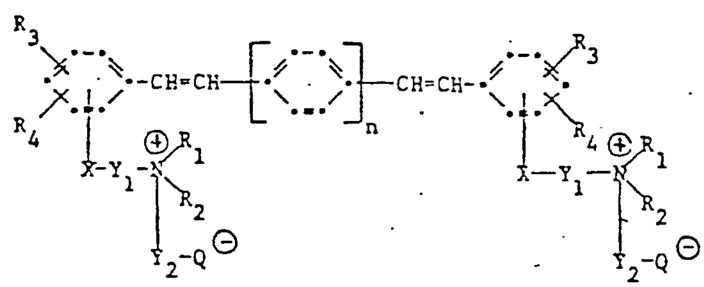

wherein

X is oxygen, sulfur, a direct bond, —$SO_2N(R_5)$—, —$CON(R_5)$— or —COO—,

$Y_1$ and $Y_2$ are each independently $C_1$-$C_4$ alkylene or hydroxypropylene,

$R_1$ and $R_2$ are each independently $C_1$-$C_4$ alkyl or, together with the N atom, are a pyrrolidine, piperidine, hexamethylene-imine or morpholine ring, and $R_1$ together with $R_5$ is also a piperazine ring,

$R_3$ and $R_4$ are hydrogen, $C_{1-4}$ alkyl, chlorine, $C_{1-4}$ alkoxy or $C_{3-4}$ alkenyl or together, in the o-position relative to each other, are a trimethylene or tetramethylene group,

$R_5$ is hydrogen, $C_{1-4}$ alkyl or cyanoethyl or, together with $R_1$, is a piperazine ring,

Q is —COO or —$SO_3$, and

n is 1 or 2.

2. A detergent composition according to claim 1, which contains an amphoteric styrene derivative of the formula

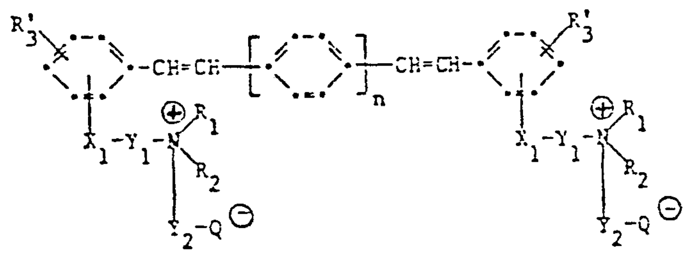

wherein

$R_1$, $R_2$, $Y_1$, $Y_2$, Q and n are as defined in claim 1,

$X_1$ is oxygen, a direct bond, —CONH— or —COO— and

$R_3'$ is hydrogen, $C_{1-4}$ alkyl, methoxy or chlorine.

3. A detergent composition according to claim 2, which contains an amphoteric styrene derivative of the formula

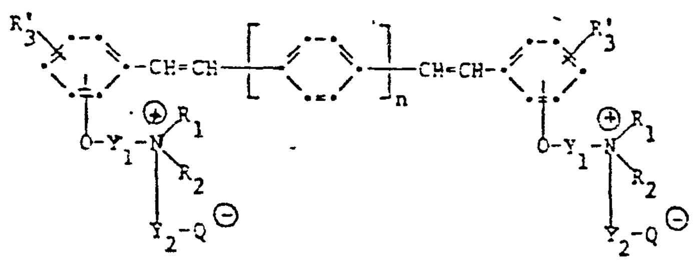

wherein $R_1$, $R_2$, $R_3'$, $Y_1$, $Y_2$, Q and n are as defined in claim 2.

4. A detergent composition according to claim 3, which contains an amphoteric styrene derivative of the formula

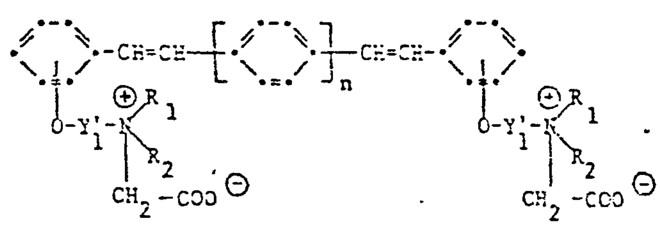

wherein

$Y_1'$ is $C_{1-4}$ alkylene and

$R_1$, $R_2$ and n are as defined in claim 3.

5. A detergent composition according to claim 4, which contains the amphoteric styrene derivative of the formula

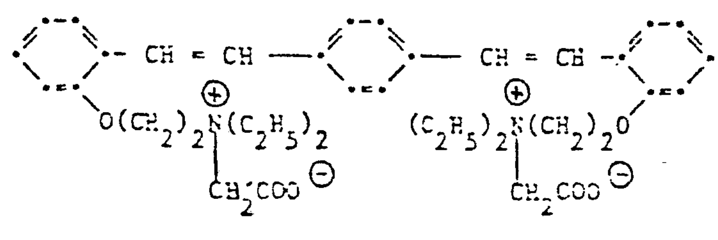

6. A detergent composition according to claim 1, which contains 10 to 70 % by weight of a non-ionic surfactant and 1 to 30 % by weight of a cationic textile softener.

7. A detergent composition according to claim 6, which contains 10 to 70 % by weight of a non-ionic surfactant and 1 to 30 % by weight of a quaternary derivative of ammonia and/or of imidazoline, having in each case 2 long-chain aliphatic radicals, as the cationic textile softener, and a solvent to bring the detergent into the liquid form.

8. The use of one or more amphoteric styrene derivatives of the formula (1) as defined in claim 1 for whitening organic materials.

9. The use according to claim 8 for whitening cellulose or polyacrylonitrile as organic material.

10. A process for the preparation of an amphoteric styrene derivative of the formula

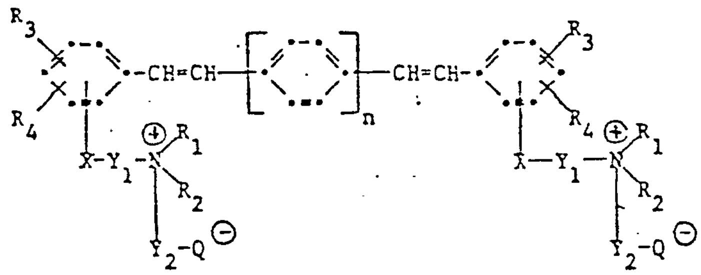

wherein

X is oxygen, sulfur, a direct bond, $-SO_2N(R_5)-$, $-CON(R_5)-$ or $-COO-$,

$Y_1$ and $Y_2$ are each independently $C_1$-$C_4$ alkylene or hydroxypropylene,

$R_1$ and $R_2$ are each independently $C_1$-$C_4$ alkyl or, together with the N atom, are a pyrrolidine, piperidine, hexamethylene-imine or morpholine ring, and $R_1$ together with $R_5$ is also a piperazine ring,

$R_3$ and $R_4$ are hydrogen, $C_{1-4}$ alkyl, chlorine, $C_{1-4}$ alkoxy or $C_{3-4}$ alkenyl or together, in the o-position relative to each other, are a trimethylene or tetramethylene group,

$R_5$ is hydrogen, $C_{1-4}$ alkyl or cyanoethyl or, together with $R_1$, is a piperazine ring,

Q is $-COO$ or $-SO_3$, and

n is 1 or 2,

which process comprises reacting a styrene derivative of the formula

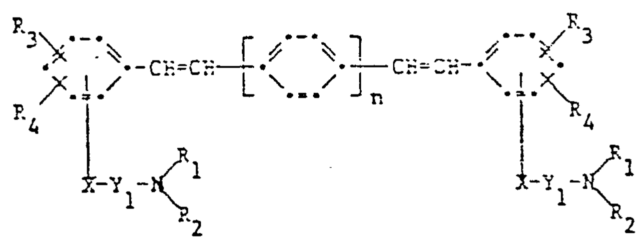

wherein $R_1$, $R_2$, $R_3$, $R_4$, X, $Y_1$ and n are as defined above, with a halide of the formula

$$Hal-Y_2-QH$$

in which $Y_2$ and Q are as defined above and Hal is chlorine or bromine, or, if Q = $-COO$, with a salt, ester, amide, nitrile or lactone thereof, or, if $Y_2$ = hydroxypropylene, also with a corresponding epoxide, and saponifying, any quaternary ammonium halide formed.

**Revendications** (pour les Etats de la Convention BE CH DE FR GB IT LI NL SE)

1. Dérivés amphotères du styrène, correspondant à la formule

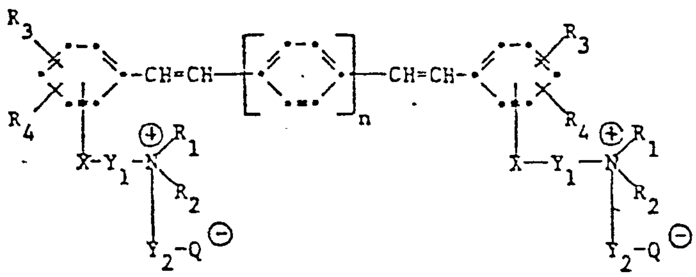

(1)

dans laquelle

X est un atome d'oxygène, de soufre, la liaison directe, $—SO_2N(R_5)—$, $—CON(R_5)—$ ou —COO—,

$Y_1$ et $Y_2$ indépendamment l'un de l'autre sont des groupes alkylène en $C_1$-$C_4$ ou hydroxypropylène,

$R_1$ et $R_2$ indépendamment l'un de l'autre sont des groupes alkyle en $C_1$-$C_4$ ou forment ensemble avec l'atome N un noyau pyrrolidine, pipéridine, hexaméthylénimine ou morpholine et $R_1$ forme avec $R_5$ également un noyau pipérazine,

$R_3$ et $R_4$ sont des atomes d'hydrogène, des groupes alkyle en $C_1$-$C_4$, des atomes de chlore, des groupes alcoxy en $C_1$-$C_4$, alcényle en $C_3$-$C_4$ ou en position ortho l'un par rapport à l'autre, forment ensemble un groupe triméthylène ou tétraméthylène,

$R_5$ est un atome d'hydrogène, un groupe alkyle en $C_1$-$C_4$, cyanéthyle ou forme avec $R_1$ un noyau pipérazine,

Q est —COO ou $—SO_3$, et

n est le nombre 1 ou 2.

2. Dérivés amphotères de styrène selon la revendication 1, ayant la formule

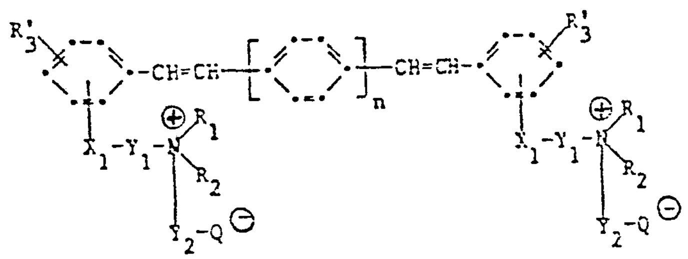

(2)

dans laquelle

$R_1$, $R_2$, $Y_1$, $Y_2$, Q et n ont les significations données dans la revendication 1, et

$X_1$ est un atome d'oxygène, la liaison directe, —CONH— ou —COO—, et

$R_3'$ est un atome d'hydrogène, un groupe alkyle en $C_1$-$C_4$, méthoxy ou un atome de chlore.

3. Dérivés amphotères de styrène selon la revendication 2, ayant la formule :

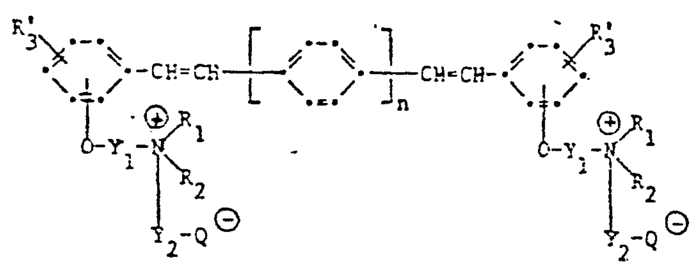

(3)

dans laquelle $R_1$, $R_2$, $R_3'$, $Y_1$, $Y_2$, Q et n ont les significations données dans la revendication 2.

4. Dérivés amphotères de styrène selon la revendication 3, ayant la formule :

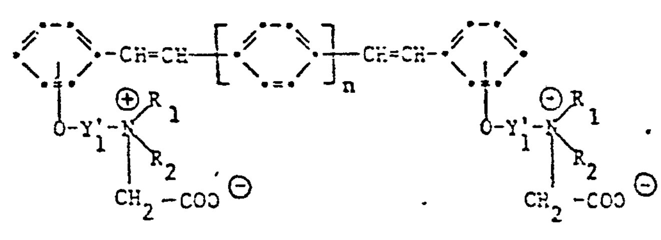

(4)

dans laquelle

$Y_1'$ est un groupe alkylène en $C_1$-$C_4$, et

$R_1$, $R_2$ et n ont les significations données dans la revendication 3.

5. Dérivés amphotères de styrène selon la revendication 4, ayant la formule :

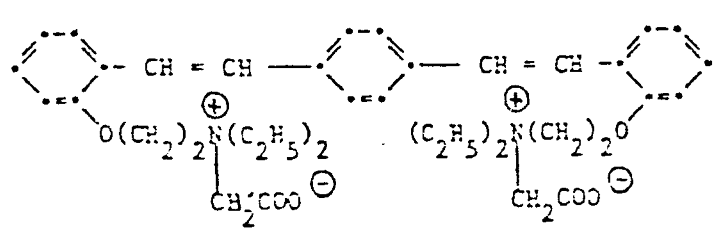

(403)

6. Procédé pour la fabrication de dérivés amphotères de styrène, ayant la formule :

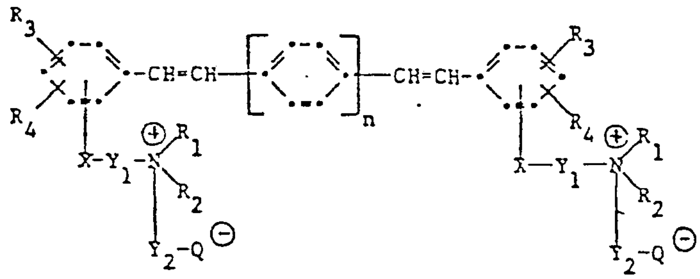

(1)

dans laquelle

X est un atome d'oxygène, de soufre, la liaison directe, —$SO_2N(R_5)$—, —$CON(R_5)$— ou —COO—,

$Y_1$ et $Y_2$ indépendamment l'un de l'autre sont des groupes alkylène en $C_1$-$C_4$ ou hydroxypropylène,

$R_1$ et $R_2$ indépendamment l'un de l'autre sont des groupes alkyle en $C_1$-$C_4$ ou forment ensemble avec l'atome N un noyau pyrrolidine, pipéridine, hexaméthylénimine ou morpholine et $R_1$ forme avec $R_5$ également un noyau pipérazine,

$R_3$ et $R_4$ sont des atomes d'hydrogène, des groupes alkyle en $C_1$-$C_4$, des atomes de chlore, des groupes alcoxy en $C_1$-$C_4$, alcényle en $C_3$-$C_4$ ou, en position ortho l'un par rapport à l'autre, forment ensemble un groupe triméthylène ou tétraméthylène,

$R_5$ est un atome d'hydrogène, un groupe alkyle en $C_1$-$C_4$, cyanéthyle ou forme avec $R_1$ un noyau pipérazine,

Q est —COO ou —$SO_3$, et

n est le nombre 1 ou 2,

caractérisé par le fait qu'on fait réagir un dérivé du styrène de formule

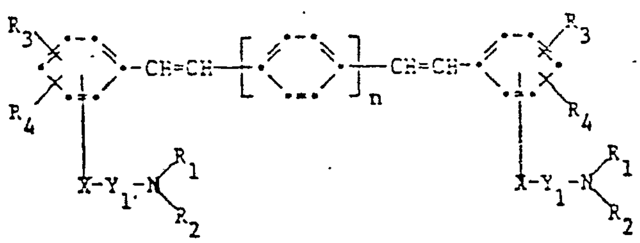

(5)

dans laquelle $R_1$, $R_2$, $R_3$, $R_4$, X, $Y_1$ et n ont les significations données ci-dessus, avec un halogénure de formule :

$$Hal—Y_2—QH$$

dans laquelle $Y_2$ et Q ont les significations indiquées ci-dessus, et Hal représente le chlore ou le brome, ou bien si Q = —COO, avec ses sels, ses esters, ses amides, ses nitriles ou ses lactones, ou bien si Q = —$SO_3$ avec ses sels, ses esters aryliques ou ses sultones, ou bien si $Y_2$ = un groupe hydroxypropylène avec également les époxydes correspondants, et on saponifie dans tous les cas les halogénures d'ammonium quaternaires qui se sont formés.

7. Utilisation des dérivés amphotères de styrène de formule (1) définie dans la revendication 1, pour l'azurage optique de matières organiques.

8. Procédé pour l'azurage optique de matières organiques, caractérisé par le fait qu'on incorpore à ces matières, ou qu'on applique sur leur surface, des dérivés amphotères de styrène de formule (1) définie dans la revendication (1).

9. Procédé selon la revendication 8, pour l'azurage optique de cellulose ou de polyacrylonitrile comme matières organiques.

10. Agent de lavage contenant un ou plusieurs dérivés amphotères de styrène de formule (1) définie dans la revendication 1.

11. Agent de lavage selon la revendication 10, qui contient 10 à 70 % d'un tensio-actif non ionique et 1 à 30 % d'un adoucisseur cationique pour textile.

12. Agent de lavage selon la revendication 11, qui contient 10 à 70 % en poids d'un tensio-actif non ionique et 1 à 30 % en poids d'un dérivé quaternaire de l'ammoniac et/ou de l'imidazoline ayant chacun deux restes aliphatiques à longue chaîne, comme adoucisseur cationique pour textile, et un solvant pour lui donner la forme liquide.

13. Agent de traitement des textiles contenant un ou plusieurs dérivés amphotères de styrène de formule (1) définie dans la revendication 1.

14. Agent de post-traitement du linge blanc contenant un ou plusieurs dérivés amphotères de styrène de formule (1) définie dans la revendication 1, et un adoucisseur cationique pour textile.

**Revendications** (pour l'état de la convention AT)

1. Agent de lavage contenant un dérivé amphotère de styrène ayant la formule :

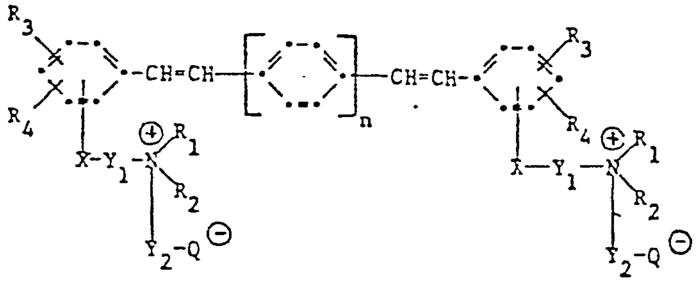

dans laquelle

X est un atome d'oxygène, de soufre, la liaison directe, $—SO_2N(R_5)—$, $—CON(R_5)—$ ou —COO—,

$Y_1$ et $Y_2$ indépendamment l'un de l'autre sont des groupes alkylène en $C_1$-$C_4$ ou hydroxypropylène,

$R_1$ et $R_2$ indépendamment l'un de l'autre sont des groupes alkyle en $C_1$-$C_4$ ou forment ensemble avec l'atome N un noyau pyrrolidine, pipéridine, hexaméthylénimine ou morpholine et $R_1$ forme ensemble avec $R_5$ également un noyau pipérazine,

$R_3$ et $R_4$ sont des atomes d'hydrogène, des groupes alkyle en $C_1$-$C_4$, des atomes de chlore, des groupes alcoxy en $C_1$-$C_4$, alcényle en $C_3$-$C_4$ ou, en position ortho l'un par rapport à l'autre, forment ensemble un groupe triméthylène ou tétraméthylène,

$R_5$ est un atome d'hydrogène, un groupe alkyle en $C_1$-$C_4$, cyanéthyle, ou forme ensemble avec $R_1$ un noyau pipérazine,

Q est —COO ou $—SO_3$ et

n est le nombre 1 ou 2.

2. Agent de lavage selon la revendication 1, contenant un dérivé amphotère de styrène ayant la formule :

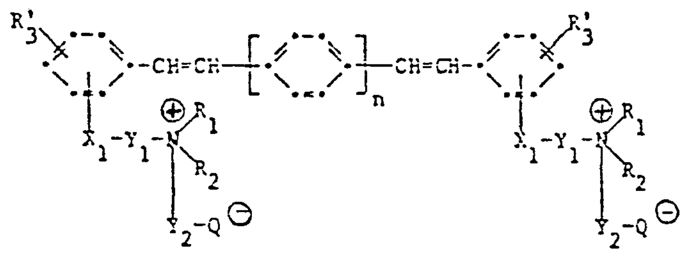

dans laquelle

$R_1$, $R_2$, $Y_1$, $Y_2$, Q et n ont les significations données dans la revendication 1, et

$X_1$ est un atome d'oxygène, la liaison directe, —CONH— ou —COO—, et

$R_3'$ est un atome d'hydrogène, un groupe alkyle en $C_1$-$C_4$, méthoxy ou un atome de chlore.

3. Agent de lavage selon la revendication 2, contenant un dérivé amphotère de styrène ayant la formule :

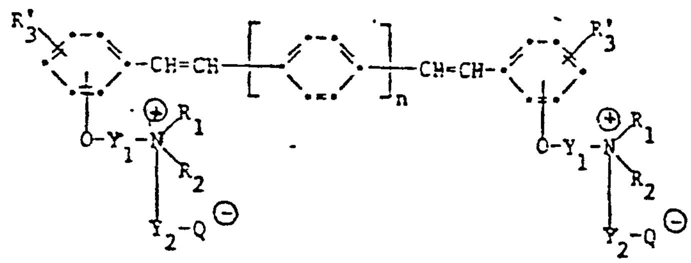

dans laquelle $R_1$, $R_2$, $R_3'$, $Y_1$, $Y_2$, Q et n ont les significations données dans la revendication 2.

4. Agent de lavage selon la revendication 3, contenant un dérivé amphotère de styrène ayant la formule :

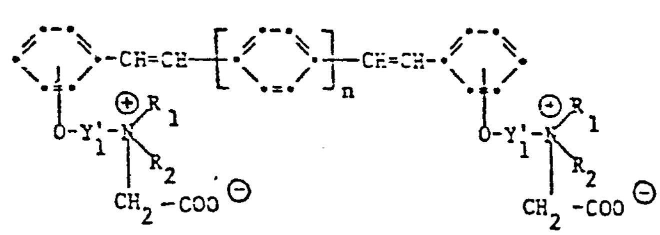

dans laquelle

$Y_1'$ est un groupe alkylène en $C_1$-$C_4$, et

$R_1$, $R_2$ et n ont les significations données dans la revendication 3.

5. Agent de lavage selon la revendication 4, contenant le dérivé amphotère de styrène ayant la formule :

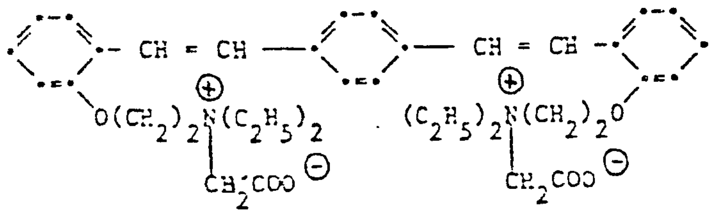

6. Agent de lavage selon la revendication 1, qui contient encore 10 à 70 % en poids de tensio-actif non ionique et 1 à 30 % d'un adoucisseur cationique pour textiles.

7. Agent de lavage selon la revendication 6, qui contient 10 à 70 % en poids d'un tensio-actif non ionique et 1 à 30 % en poids d'un dérivé quaternaire de l'ammoniac et/ou de l'imidazoline ayant chacun 2 restes aliphatiques à longue chaîne, comme adoucisseur cationique pour textiles, et un solvant pour lui donner la forme liquide.

8. Utilisation de un ou de plusieurs dérivés amphotères de styrène de formule (1), définis dans la revendication 1, pour l'azurage optique de matières organiques.

9. Utilisation selon la revendication 8 pour l'azurage optique de cellulose ou de polyacrylonitrile comme matières organiques.

10. Procédé pour la préparation de dérivés amphotères de styrène de formule :

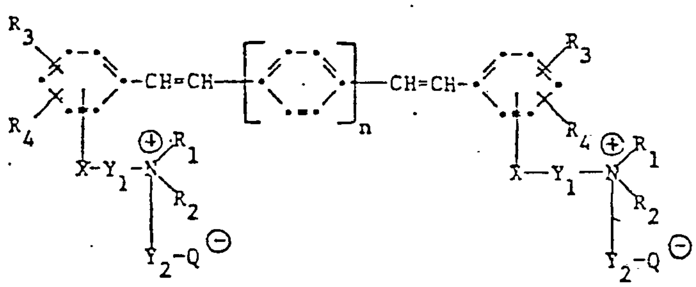

dans laquelle

X est un atome d'oxygène, de soufre, la liaison directe, $—SO_2N(R_5)—$, $—CON(R_5)—$ ou —COO—,

$Y_1$ et $Y_2$ indépendamment l'un de l'autre, sont des groupes alkylène en $C_1$-$C_4$ ou hydroxypropylène,

$R_1$ et $R_2$ indépendamment l'un de l'autre sont des groupes alkyle en $C_1$-$C_4$ ou forment ensemble avec l'atome N un noyau pyrrolidine, pipéridine, hexaméthylénimine, ou morpholine, et $R_1$ forme avec $R_5$ également un noyau pipérazine,

$R_3$ et $R_4$ sont des atomes d'hydrogène, des groupes alkyle en $C_1$-$C_4$, des atomes de chlore, des groupes alcoxy en $C_1$-$C_4$, alcényle en $C_3$-$C_4$ ou, en position ortho l'un par rapport à l'autre, forment ensemble un groupe triméthylène ou tétraméthylène,

$R_5$ est un atome d'hydrogène, un groupe alkyle en $C_1$-$C_4$, cyanéthyle, ou forme avec $R_1$ un noyau pipérazine,

Q est —COO ou $—SO_3$, et

n est le nombre 1 ou 2,

caractérisé par le fait qu'on fait réagir un dérivé du styrène de formule :

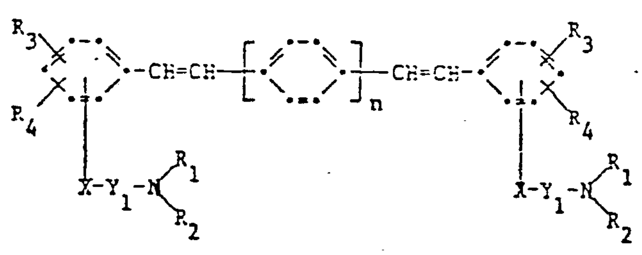

dans laquelle $R_1$, $R_2$, $R_3$, $R_4$, X, $Y_1$ et n ont les significations données ci-dessus, avec un halogénure de formule :

$$Hal—Y_2—QH$$

dans laquelle $Y_2$ et Q ont les significations données ci-dessus, et Hal représente le chlore ou le brome, ou bien si Q = —COO, avec ses sels, ses esters, ses amides, ses nitriles ou ses lactones ou bien si Q = —$SO_3$, avec ses sels, ses esters aryliques ou ses sultones ou bien si $Y_2$ = un groupe hydroxypropylène également avec les époxydes correspondants, et on saponifie dans tous les cas les hélogénures d'ammonium quaternaires qui se sont formés.